(19) Europäisches Patentamt
European Patent Office
Office européen des brevets

(11) **EP 1 520 861 A1**

(12) **EUROPEAN PATENT APPLICATION**

(43) Date of publication:
**06.04.2005 Bulletin 2005/14**

(51) Int Cl.[7]: **C07K 14/705**

(21) Application number: **03020430.9**

(22) Date of filing: **11.09.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Aventis Pharma Deutschland GmbH**
**65929 Frankfurt am Main (DE)**

(72) Inventors:
- **Arndt, Petra, Dr.**
  **(DE)**
- **Margerie, Daniel, Dr.**
  **60486 Frankfurt am Main (DE)**
- **Muench, Götz, Dr.**
  **80799 München (DE)**
- **Ungerer, Martin, Dr.**
  **80799 München (DE)**

(54) **Test system for the identification of APJ receptor ligands**

(57) The present invention refers to a test system for the identification of a ligand for angiotension receptor like-1 (APJ receptor) comprising an APJ receptor or a functional variant thereof and a Gαq or a GαI protein or a hybrid protein which is assembled from portions of different G proteins like Gαq or GαI proteins, a method of screening an APJ receptor ligand using the test system, a method for producing a medicament, a method for diagnosis and prognosis of a heart disease and a kit usable for detection or prognosis of a heart disease.

EP 1 520 861 A1

SEQUENCE LISTING

<110> Aventis Pharma Deutschland GmbH

<120> Test System for the Identification of APJ Receptor Ligands

<130> DEAV 2003/0066

<140>
<141>

<160> 8

<170> PatentIn Ver. 2.1

<210> 1
<211> 1080
<212> DNA
<213> Artificial Sequence

<220>
<223> Description of Artificial Sequence:Artificial

<400> 1
bamhggatcc atggggtgct gcctgagcga ggaggccaag gaagcccggc ggatcaacga 60
cgagatcgag cggcacgtcc gcagggacaa gcgggacgcc cgccgggagc tcaagctgct 120
gctgctcggg acaggagaga gtggcaagag tacgtttatc aagcagatga gaatcatcca 180
tgggtcagga tactctgatg aagataaaag ggcttcacc aagctggtgt atcagaacat 240
cttcacggcc atgcaggcca tgatcagagc catggacaca ctcaagatcc catacaagta 300
tgagcacaat aaggctcatg cacaattagt tcgagaagtt gatgtggaga aggtgtctgc 360
ttttgacgtc cccgactacg cggcaataaa gagtttatgg aatgatcctg gaatccagga 420
atgctatgat agacgacgag aatatcaatt atctgactct accaaatact atcttaatga 480
cttggaccgc gtagctgacc ctgcctacct gcctacgcaa caagatgtgc ttagagttcg 540
agtccccacc acagggatca tcgaataccc ctttgactta caaagtgtca ttttcagaat 600
ggtcgatgta gggggccaaa ggtcagagag aagaaaatgg atacactgct ttgaaaatgt 660
cacctctatc atgtttctag tagcgcttag tgaatatgat caagttctcg tggagtcaga 720
caatgagaac cgaatggagg aaagcaaggc tctctttaga acaattatca catacccctg 780
gttccagaac tcctcggtta ttctgttctt aaacaagaaa gatcttctag aggagaaaat 840
catgtattcc catctagtcg actacttccc agaatatgat ggaccccaga gagatgccca 900
ggcagcccga gaattcattc tgaagatgtt cgtggacctg aacccagaca gtgacaaaat 960
tatctactcc cacttcacgt gcgccacaga caccgagaat atccgctttg tctttgctgc 1020
cgtcaaggac accatcctcc agttgaacct gaaggagtgt ggcctcttct aaatgcatns 1080

<210> 2
<211> 14988
<212> DNA

<213> Artificial Sequence

<220>
<223> Description of Artificial Sequence:Artificial

<400> 2
ccaccgctac cagcggtggt ttgtttgccg gatcaagagc taccaactct ttttccgaag 60
ggtggcgatg gtcgccacca aacaaacggc ctagttctcg atggttgaga aaaaggcttc 120
gtaactggct tcagcagagc gcagatacca aatactgtcc ttctagtgta gccgtagtta 180
cattgaccga agtcgtctcg cgtctatggt ttatgacagg aagatcacat cggcatcaat 240
ggccaccact tcaagaactc tgtagcaccg cctacatacc tcgctctgct aatcctgtta 300
ccggtggtga agttcttgag acatcgtggc ggatgtatgg agcgagacga ttaggacaat 360
ccagtggctg ctgccagtgg cgataagtcg tgtcttaccg ggttggactc aagacgatag 420
ggtcaccgac gacggtcacc gctattcagc acagaatggc ccaacctgag ttctgctatc 480
ttaccggata aggcgcagcg gtcgggctga acggggggtt cgtgcacaca gcccagcttg 540
aatggcctat tccgcgtcgc cagcccgact tgcccccaa gcacgtgtgt cgggtcgaac 600
gagcgaacga cctacaccga actgagatac ctacagcgtg agcattgaga aagcgccacg 660
ctcgcttgct ggatgtggct tgactctatg gatgtcgcac tcgtaactct ttcgcggtgc 720
cttcccgaag ggagaaaggc ggacaggtat ccggtaagcg gcagggtcgg aacaggagag 780
gaagggcttc cctctttccg cctgtccata ggccattcgc cgtcccagcc ttgtcctctc 840
cgcacgaggg agcttccagg gggaaacgcc tggtatcttt atagtcctgt cgggtttcgc 900
gcgtgctccc tcgaaggtcc ccctttgcgg accatagaaa tatcaggaca gcccaaagcg 960
cacctctgac ttgagcgtcg atttttgtga tgctcgtcag gggggcggag cctatggaaa 1020
gtggagactg aactcgcagc taaaaacact acgagcagtc cccccgcctc ggataccttt 1080
aacgccagca acgcaagcta gagtttaaac ttgacagatg agacaataac cctgataaat 1140
ttgcggtcgt tgcgttcgat ctcaaatttg aactgtctac tctgttattg ggactattta 1200
gcttcaataa tattgaaaaa ggaaaagtat gagtattcaa catttccgtg tcgcccttat 1260
cgaagttatt ataacttttt ccttttcata ctcataagtt gtaaaggcac agcgggaata 1320
tccctttttt gcggcatttt gccttcctgt ttttgctcac ccagaaacgc tggtgaaagt 1380
agggaaaaaa cgccgtaaaa cggaaggaca aaaacgagtg ggtctttgcg accactttca 1440
aaaagatgca gaagatcact tgggtgcgcg agtgggttac atcgaactgg atctcaacag 1500
ttttctacgt cttctagtga acccacgcgc tcacccaatg tagcttgacc tagagttgtc 1560
cggtaagatc cttgagagtt ttcgccccga agaacgtttc ccaatgatga gcacttttaa 1620
gccattctag gaactctcaa aagcggggct tcttgcaaag ggttactact cgtgaaaatt 1680
agttctgcta tgtggcgcgg tattatcccg tattcttggt tgaatactca ccagtcacag 1740
tcaagacgat acaccgcgcc ataatagggc ataagaacca acttatgagt ggtcagtgtc 1800
aaaagcatct tacggatggc atgacagtaa gagaattatg cagtgctgcc ataaccatga 1860
ttttcgtaga atgcctaccg tactgtcatt ctcttaatac gtcacgacgg tattggtact 1920
gtgataacac tgcggccaac ttacttctga caactatcgg aggaccgaag gagctaaccg 1980
cactattgtg acgccggttg aatgaagact gttgatagcc tcctggcttc ctcgattggc 2040
ctttttgca caacatgggg gatcatgtaa ctcgccttga tcgttgggaa ccggagctga 2100
gaaaaaacgt gttgtacccc ctagtacatt gagcggaact agcaaccctt ggcctcgact 2160
atgaagccat accaaacgac gagcgtgaca ccacgatgcc tgtagcaatg gcaacaacgt 2220
tacttcggta tggtttgctg ctcgcactgt ggtgctacgg acatcgttac cgttgttgca 2280
tgcgaaaact attaactggc gaactactta ctctagcttc ccggcaacaa ctaatagact 2340
acgcttttga taattgaccg cttgatgaat gagatcgaag ggccgttgtt gattatctga 2400
ggatggaggc ggataaagtt gcaggaccac ttctgcgctc ggcacttccg gctggctggt 2460
cctacctccg cctatttcaa cgtcctggtg aagacgcgag ccgtgaaggc cgaccgacca 2520

ttattgctga taaatcagga gccggtgagc gtgggtcacg cggtatcatt gcagcactgg 2580
aataacgact atttagtcct cggccactcg cacccagtgc gccatagtaa cgtcgtgacc 2640
ggccggatgg taagccctcc cgtatcgtag ttatctacac tacggggagt caggcaacta 2700
ccggcctacc attcgggagg gcatagcatc aatagatgtg atgcccctca gtccgttgat 2760
tggatgaacg aaatagacag atcgctgaga taggtgcctc actgattaag cattggtaag 2820
acctacttgc tttatctgtc tagcgactct atccacggag tgactaattc gtaaccattc 2880
gataaatttc tggtaaggag gacacgtatg gaagtgggca agttggggaa gccgtatccg 2940
ctatttaaag accattcctc ctgtgcatac cttcacccgt tcaacccctt cggcataggc 3000
ttgctgaatc tggcatatgt gggagtataa gacgcgcagc gtcgcatcag gcattttttt 3060
aacgacttag accgtataca ccctcatatt ctgcgcgtcg cagcgtagtc cgtaaaaaaa 3120
ctgcgccaat gcaaaaaggc catccgtcag gatggccttt cgcataacta gtgaggctcc 3180
gacgcggtta cgtttttccg gtaggcagtc ctaccggaaa gcgtattgat cactccgagg 3240
ggtgcccgtc agtgggcaga gcgcacatcg cccacagtcc ccgagaagtt ggggggaggg 3300
ccacgggcag tcacccgtct cgcgtgtagc gggtgtcagg ggctcttcaa ccccccctccc 3360
gtcggcaatt gaaccggtgc ctagagaagg tggcgcgggg taaactggga aagtgatgtc 3420
cagccgttaa cttggccacg gatctcttcc accgcgcccc atttgaccct ttcactacag 3480
gtgtactggc tccgcctttt tcccgagggt gggggagaac cgtatataag tgcagtagtc 3540
cacatgaccg aggcggaaaa agggctccca cccccctcttg gcatatattc acgtcatcag 3600
gccgtgaacg ttctttttcg caacgggttt gccgccagaa cacaggtaag tgccgtgtgt 3660
cggcacttgc aagaaaaagc gttgcccaaa cggcggtctt gtgtccattc acggcacaca 3720
ggttcccgcg ggcctggcct ctttacgggt tatggccctt gcgtgccttg aattacttcc 3780
ccaagggcgc ccggaccgga gaaatgccca ataccgggaa cgcacggaac ttaatgaagg 3840
acctggctgc agtacgtgat tcttgatccc gagcttcggg ttggaagtgg gtgggagagt 3900
tggaccgacg tcatgcacta agaactaggg ctcgaagccc aaccttcacc caccctctca 3960
tcgaggcctt gcgcttaagg agccccttcg cctcgtgctt gagttgaggc ctggcctggg 4020
agctccggaa cgcgaattcc tcggggaagc ggagcacgaa ctcaactccg gaccggaccc 4080
cgctggggcc gccgcgtgcg aatctggtgg caccttcgcg cctgtctcgc tgctttcgat 4140
gcgaccccgg cggcgcacgc ttagaccacc gtggaagcgc ggacagagcg acgaaagcta 4200
aagtctctag ccatttaaaa tttttgatga cctgctgcga cgcttttttt ctggcaagat 4260
ttcagagatc ggtaaatttt aaaaactact ggacgacgct gcgaaaaaaa gaccgttcta 4320
agtcttgtaa atgcgggcca agatcgatct gcacactggt atttcggttt ttggggccgc 4380
tcagaacatt tacgcccggt tctagctaga cgtgtgacca taaagccaaa aaccccggcg 4440
gggcggcgac ggggcccgtg cgtcccagcg cacatgttcg gcgaggcggg gcctgcgagc 4500
cccgccgctg ccccgggcac gcagggtcgc gtgtacaagc cgctccgccc cggacgctcg 4560
gcggccaccg agaatcggac gggggtagtc tcaagctggc cggcctgctc tggtgcctgg 4620
cgccggtggc tcttagcctg cccccatcag agttcgaccg gccggacgag accacggacc 4680
cctcgcgccg ccgtgtatcg ccccgccctg ggcggcaagg ctggcccggt cggcaccagt 4740
ggagcgcggc ggcacatagc ggggcgggac ccgccgttcc gaccgggcca gccgtggtca 4800
tgcgtgagcg gaaagatggc cgcttcccgg ccctgctgca gggagctcaa aatggaggac 4860
acgcactcgc ctttctaccg gcgaagggcc gggacgacgt ccctcgagtt ttacctcctg 4920
gcggcgctcg ggagagcggg cgggtgagtc acccacacaa aggaaaaggg cctttccgtc 4980
cgccgcgagc cctctcgccc gcccactcag tgggtgtgtt tccttttccc ggaaaggcag 5040
ctcagccgtc gcttcatgtg actccacgga gtaccgggcg ccgtccaggc acctcgatta 5100
gagtcggcag cgaagtacac tgaggtgcct catggcccgc ggcaggtccg tggagctaat 5160
hgttctcgag cttttggagt acgtcgtctt taggttgggg ggaggggttt tatgcgatgg 5220
acaagagctc gaaaacctca tgcagcagaa atccaacccc cctccccaaa atacgctacc 5280
tdragtttcc ccacactgag tgggtggaga ctgaagttag gccagcttgg cacttgatgt 5340
aattcaaagg ggtgtgactc acccacctct gacttcaatc cggtcgaacc gtgaactaca 5400

- ttaactcctt ggaatttgcc ctttttgagt ttggatcttg gttcattctc aagcctcaga 5460
cagtgaggaa ccttaaacgg gaaaaactca aacctagaac caagtaagag ttcggagtct 5520
gtcahndggt tcaaagtttt tttcttccat ttcaggtgtc gtgaaaagct tgccaccatg 5580
gactacacca agtttcaaaa aaagaaggta aagtccacag cacttttcga acggtggtac 5640
ctgatgtrcn tycraggacg acgacgacaa gaacatgtgg ggcgaattaa ttccttaagc 5700
tatcaacaag tttgtcctgc tgctgctgtt cttgtacacc ccgcttaatt aaggaattcg 5760
atagttgttc aaactacaaa aaagcaggct taggaatgga ggaaggtggt gattttgaca 5820
actactatgg ggcaatgttt tttcgtccga atccttacct ccttccacca ctaaaactgt 5880
tgatgatacc ccgtmggddn yygagacaac cagtctgagt gtgagtacac agactggaaa 5940
tcctcggggg ccctcatccc tgccctgttg gtcagactca cactcatgtg tctgaccttt 6000
aggagccccc gggagtaggg acggdnscyt dwkssgaaat ctacatgttg gtcttcctcc 6060
tgggcaccac gggaaacggt ctggtgctct ggaccgtgta gatgtacaac cagaaggagg 6120
acccgtggtg ccctttgcca gaccacgaga cctggcacym vgttgngvwt vcrvtttcgg 6180
agcagccggg agaagaggcg ctcagctgat atcttcattg ctagcctggc ggtgaaagcc 6240
tcgtcggccc tcttctccgc gagtcgacta tagaagtaac gatcggaccg ccacrssrkr 6300
rsadasavgc tgacctgacc ttcgtggtga cgctgcccct gtgggctacc tacacgtacc 6360
gggactatcg actggactgg aagcaccact gcgacgggga cacccgatgg atgtgcatgg 6420
ccctgataad tvvtwatyty rdygactggc cctttgggac cttcttctgc aagctcagca 6480
gctacctcat cttcgtcaac atgctgaccg ggaaaccctg gaagaagacg ttcgagtcgt 6540
cgatggagta gaagcagttg tacdwgtcks syvnmtacgc cagcgtcttc tgcctcaccg 6600
gcctcagctt cgaccgctac ctggccatcg tgaggatgcg gtcgcagaag acggagtggc 6660
cggagtcgaa gctggcgatg gaccggtagc actccyasvc tgsdryavrc cagtggccaa 6720
tgctcggctg aggctgcggg tcagcggggc cgtggccacg gcagttcttg gtcaccggtt 6780
acgagccgac tccgacgccc agtcgccccg gcaccggtgc cgtcaagaav anarrrvsga 6840
vatavtgggt gctggccgcc ctcctggcca tgcctgtcat ggtgttacgc accaccgggg 6900
acttgaccca cgaccggcgg gaggaccggt acggacagta ccacaatgcg tggtggcccc 6960
tgaacwvaaa mvmvrttgdg agaacaccac taaggtgcag tgctacatgg actactccat 7020
ggtggccact gtgagctcac tcttgtggtg attccacgtc acgatgtacc tgatgaggta 7080
ccaccggtga cactcgagtn ttkvcymdys mvatvssgag tgggcctggg aggtgggcct 7140
tggggtctcg tccaccaccg tgggctttgt ggtgcccctc acccggaccc tccacccgga 7200
accccagagc aggtggtggc acccgaaaca ccacgggwaw vggvssttvg vvttcaccat 7260
catgctgacc tgttacttct tcatcgccca aaccatcgct ggccacttcc gcaagtggta 7320
gtacgactgg acaatgaaga agtagcgggt ttggtagcga ccggtgaagg cgtmtcyata 7380
ghraaggaac gcatcgaggg cctgcggaag cggcgccggc tgctcagcat catcgtggtg 7440
ctgttccttg cgtagctccc ggacgccttc gccgcggccg acgagtcgta gtagcaccac 7500
gackrgrkrr rsvvdragtg gtgacctttg ccctgtgctg gatgccctac cacctggtga 7560
agacgctgta catgctgcac cactggaaac gggacacgac ctacgggatg gtggaccact 7620
tctgcgacat gtacgacvvt acwmyhvkty mggcagcctg ctgcactggc cctgtgactt 7680
tgacctcttc ctcatgaaca tcttccccta cccgtcggac gacgtgaccg ggacactgaa 7740
actggagaag gagtacttgt agaaggggat ggshwcddmn ytgcacctgc atcagctacg 7800
tcaacagctg cctcaacccc ttcctctatg cctttttcga cacgtggacg tagtcgatgc 7860
agttgtcgac ggagttgggg aaggagatac ggaaaaagct gctcsyvnsc nyadccccgc 7920
ttccgccagg cctgcacctc catgctctgc tgtggccaga gcaggtgcgc aggcggggcg 7980
aaggcggtcc ggacgtggag gtacgagacg acaccggtct cgtccacgcg tccgrracts 8040
mccgsrcaga cctcccacag cagcagtggg gagaagtcag ccagctactc ttcggggcac 8100
agccaggggt ggagggtgtc gtcgtcaccc ctcttcagtc ggtcgatgag aagccccgtg 8160
tcggtcccct shsssgksas yssghsgccc ggccccaaca tgggcaaggg tggagaacag 8220
atgcacgaga aatccatccc ctacagcggg ccggggttgt acccgttccc acctcttgtc 8280

tacgtgctct ttaggtaggg gatgtcggnm gkggmhksys caggagaccc ttgtggttga 8340
ctaggaccca gctttcttgt acaaagtggt tgatagcttg gtcctctggg aacaccaact 8400
gatcctgggt cgaaagaaca tgtttcacca actatcgaac tvvd*bamhr cntycrgtac 8460
cgagctcgga tccactagtc cagtgtggtg gaattggccg caggtaagcc agcccacatg 8520
gctcgagcct aggtgatcag gtcacaccac cttaaccggc gtccattcgg tcgggtggcc 8580
tcgccctcca gctcaaggcg ggacaggtgc cctagagtag cctgcatcca gggacaccgg 8640
agcgggaggt cgagttccgc cctgtccacg ggatctcatc ggacgtaggt ccctgtggcc 8700
ccagccgggt gctgacacgt ccacctccat ctcttcctca ggtctgcccg ggtggcccgg 8760
ggtcggccca cgactgtgca ggtggaggta gagaaggagt ccagacgggc ccaccgatcc 8820
ctgtgacccc tccccagtgc ctctcctggc cctggaagtt gccactccag tgcccatagg 8880
gacactgggg aggggtcacg gagaggaccg ggaccttcaa cggtgaggtc acgggtccag 8940
ccttgtccta ataaaattaa gttgcatcat tttgtctgac taggtgtcct tctataggtc 9000
ggaacaggat tattttaatt caacgtagta aaacagactg atccacagga agatatatat 9060
tatggggtgg agggggtgg tatggagcaa ggggcccaag ttaacttgtt tattgctata 9120
ataccccacc tccccccacc atacctcgtt ccccgggttc aattgaacaa ataacgagct 9180
tataatggtt acaaataaag caatagcatc acaaatttca caaataaagc atttttttcga 9240
atattaccaa tgtttatttc gttatcgtag tgtttaaagt gtttatttcg taaaaabamh 9300
ttcactgcat tctagttgtg gtttgtccaa actcatcaat gtatcttatc atgtctggat 9360
aagtgacgta agatcaacac caaacaggtt tgagtagtta catagaatag tacagaccta 9420
ccgcttcagg caccgggctt gcgggtcatg caccaggtcg cgcggtcctt cggcactcg 9480
ggcgaagtcc gtggcccgaa cgcccagtac gtggtccagc gcgccaggaa gcccgtgagc 9540
acgtcggcgg tgacggtgaa gccgagccgc tcgtagaagg ggaggttgcg gggcgcggag 9600
tgcagccgcc actgccactt cggctcggcg agcatcttcc cctccaacgc cccgcgcctc 9660
gtctccagga aggcgggcac cccggcgcgc tcggccgcct ccactccggg gagcacgacg 9720
cagaggtcct tccgcccgtg gggccgcgcg agccggcgga ggtgaggccc ctcgtgctgc 9780
gcgctgccca gacccttgcc ctggtggtcg ggcgagacgc cgacggtggc caggaaccac 9840
cgcgacgggt ctgggaacgg gaccaccagc ccgctctgcg gctgccaccg gtccttggtg 9900
gcgggctcct tgggccggtg cggcgccagg aggccttcca tctgttgctg cgcggccagc 9960
cgcccgagga acccggccac gccgcggtcc tccggaaggt agacaacgac gcgccggtcg 10020
cgggaaccgc tcaactcggc catgcgcggg ccgatctcgg cgaacaccgc ccccgcttcg 10080
gcccttggcg agttgagccg gtacgcgccc ggctagagcc gcttgtggcg ggggcgaagc 10140
acgctctccg gcgtggtcca gaccgccacc gcggcgccgt cgtccgcgac ccacaccttg 10200
tgcgagaggc cgcaccaggt ctggcggtgg cgccgcggca gcaggcgctg ggtgtggaac 10260
ccgatgtcga gcccgacgcg cgtgaggaag agttcttgca gctcggtgac ccgctcgatg 10320
ggctacagct cgggctgcgc gcactccttc tcaagaacgt cgagccactg ggcgagctac 10380
tggcggtccg ggtcgacggt gtggcgcgtg gcggggtagt cggcgaacgc ggcggcgagg 10440
accgccaggc ccagctgcca caccgcgcac cgccccatca gccgcttgcg ccgccgctcc 10500
gtgcgtacgg cccgggggac gtcgtcgcgg gtggcgaggc gcaccgtggg cttgtactcg 10560
cacgcatgcc gggccccctg cagcagcgcc caccgctccg cgtggcaccc gaacatgagc 10620
gtcatggtgg cctgcagagt cgctcggtgt tcgaggccac acgcgtcacc ttaatatgcg 10680
cagtaccacc ggacgtctca gcgagccaca agctccggtg tgcgcagtgg aattatacgc 10740
aagtggacct gggaccgcgc cgccccgact gcatctgcgt gttaattcgc caatgacaag 10800
ttcacctgga ccctggcgcg gcggggctga cgtagacgca caattaagcg gttactgttc 10860
acgctgggcg gggtttgtgt catcatagaa ctaaagacat gcaaatatat ttcttccggg 10920
tgcgacccgc cccaaacaca gtagtatctt gatttctgta cgtttatata aagaaggccc 10980
gacaccgcca gcaaacgcga gcaacgggcc acggggatga agcagctgcg ccactccctg 11040
ctgtggcggt cgtttgcgct cgttgcccgg tgcccctact tcgtcgacgc ggtgagggac 11100
aagatccatc gtctcctaac aagttacatc actcctgccc ttcctcaccc tcatctccat 11160

ttctaggtag cagaggattg ttcaatgtag tgaggacggg aaggagtggg agtagaggta 11220
cacctccttc atctccgtca tctccgtcat caccctccgc ggcagcccct tccaccatag 11280
gtggaggaag tagaggcagt agaggcagta gtgggaggcg ccgtcgggga aggtggtatc 11340
gtggaaacca gggaggcaaa tctactccat cgtcaaagct gcacacagtc accctgatat 11400
cacctttggt ccctccgttt agatgaggta gcagtttcga cgtgtgtcag tgggactata 11460
tgcaggtagg agcgggcttt gtcataacaa ggtccttaat cgcatccttc aaaacctcag 11520
acgtccatcc tcgcccgaaa cagtattgtt ccaggaatta gcgtaggaag ttttggagtc 11580
caaatatatg agtttgtaaa aagaccatga aataacagac aatggactcc cttagcgggc 11640
gtttatatac tcaaacattt ttctggtact ttattgtctg ttacctgagg gaatcgcccg 11700
caggttgtgg gccgggtcca ggggccattc caaaggggag acgactcaat ggtgtaagac 11760
gtccaacacc cggcccaggt ccccggtaag gtttcccctc tgctgagtta ccacattctg 11820
gacattgtgg aatagcaagg gcagttcctc gccttaggtt gtaaagggag gtcttactac 11880
ctgtaacacc ttatcgttcc cgtcaaggag cggaatccaa catttccctc cagaatgatg 11940
ctccatatac gaacacaccg gcgacccaag ttccttcgtc ggtagtcctt tctacgtgac 12000
gaggtatatg cttgtgtggc cgctgggttc aaggaagcag ccatcaggaa agatgcactg 12060
tcctagccag gagagctctt aaaccttctg caatgttctc aaatttcggg ttggaacctc 12120
aggatcggtc ctctcgagaa tttggaagac gttacaagag tttaaagccc aaccttggag 12180
cttgaccacg atgctttcca aaccaccctc cttttttgcg cctgcctcca tcaccctgac 12240
gaactggtgc tacgaaaggt ttggtgggag gaaaaaacgc ggacggaggt agtgggactg 12300
ccccgctgcg cgggggcacg tcaggctcac catctgggcc gccttcttgg tggtattcaa 12360
ggggcgacgc gccccccgtgc agtccgagtg gtagacccgg cggaagaacc accataagtt 12420
aataatcggc ttcccctaca gggtggaaaa atggccttct acctggaggg ggcctgcgcg 12480
ttattagccg aaggggatgt cccacctttt taccggaaga tggacctccc ccggacgcgc 12540
gtggagaccc ggatgatgat gactgactac tgggactcct gggcctcttt tctccacgtc 12600
cacctctggg cctactacta ctgactgatg accctgagga cccggagaaa agaggtgcag 12660
cacgacctct ccccctggct ctttcacgac ttcccccccct ggctctttca cgtcctctac 12720
gtgctggaga gggggaccga gaaagtgctg aaggggggga ccgagaaagt gcaggagatg 12780
cccggcggcc tccactacct cctcgacccc ggcctccact acctcctcga ccccggcctc 12840
gggccgccgg aggtgatgga ggagctgggg ccggaggtga tggaggagct ggggccggag 12900
cactgcctcc tcgaccccgg cctccacctc ctgctcctgc ccctcccgct cctgctcctg 12960
gtgacggagg agctggggcc ggaggtggag gacgaggacg gggagggcga ggacgaggac 13020
ctcctgttcc accgtgggtc cctttgcagc caatgcaact tggacgtttt tggggtctcc 13080
gaggacaagg tggcacccag ggaaacgtcg gttacgttga acctgcaaaa accccagagg 13140
ggacaccatc tctatgtctt ggccctgatc ctgagccgcc cggggctcct ggtcttccgc 13200
cctgtggtag agatacagaa ccgggactag gactcggcgg gccccgagga ccagaaggcg 13260
ctcctcgtcc tcgtcctctt ccccgtcctc gtccatgtgc catgatggcg gcctgcagct 13320
gaggagcagg agcaggagaa ggggcaggag caggtacacg gtactaccgc cggacgtcga 13380
gtgttcgagg ccgcgcgtgt caccttaata tgcgaagtgg acctgggacc gcgccgcccc 13440
cacaagctcc ggcgcgcaca gtggaattat acgcttcacc tggaccctgg cgcggcgggg 13500
gactgcatct gcgtgttcga gttcgccaat gacaagacgc tgggcgggga gatccccctt 13560
ctgacgtaga cgcacaagct caagcggtta ctgttctgcg acccgcccct ctagggggaa 13620
attaacccta aacgggtagc atatgcttcc cgggtagtag tatatactat ccagactaac 13680
taattgggat ttgcccatcg tatacgaagg gcccatcatc atatatgata ggtctgattg 13740
cctaattcaa tagcatatgt tacccaacgg gaagcatatg ctatcgaatt agggttagta 13800
ggattaagtt atcgtataca atgggttgcc cttcgtatac gatagcttaa tcccaatcat 13860
aaagggtcct aaggaacagc gatctggata gcatatgcta tcctaatcta tatctgggta 13920
tttcccagga ttccttgtcg ctagacctat cgtatacgat aggattagat atagacccat 13980
gcatatgcta tcctaatcta tatctgggta gcataggcta tcctaatcta tatctgggta 14040

```
cgtatacgat aggattagat atagacccat cgtatccgat aggattagat atagacccat 14100
gcatatgcta tcctaatcta tatctgggta gtatatgcta tcctaattta tatctgggta 14160
cgtatacgat aggattagat atagacccat catatacgat aggattaaat atagacccat 14220
gcataggcta tcctaatcta tatctgggta gcatatgcta tcctaatcta tatctgggta 14280
cgtatccgat aggattagat atagacccat cgtatacgat aggattagat atagacccat 14340
gtatatgcta tcctaatctg tatccgggta gcatatgcta tcctcatgca tatacagtca 14400
catatacgat aggattagac ataggcccat cgtatacgat aggagtacgt atatgtcagt 14460
gcatatgata cccagtagta gagtgggagt gctatccttt gcatatgccg ccacctccca 14520
cgtatactat gggtcatcat ctcaccctca cgataggaaa cgtatacggc ggtggagggt 14580
aggagatctg tcgacatcga tgggcgcggg tgtacactcc gcccatcccg cccctaactc 14640
tcctctagac agctgtagct acccgcgccc acatgtgagg cgggtagggc ggggattgag 14700
cgcccagttc cgcccattct ccgcctcatg gctgactaat ttttttatt tatgcagagg 14760
gcgggtcaag gcgggtaaga ggcggagtac cgactgatta aaaaaaataa atacgtctcc 14820
ccgaggccgc ctcggcctct gagctattcc agaagtagtg aggaggcttt tttggaggcc 14880
ggctccggcg gagccggaga ctcgataagg tcttcatcac tcctccgaaa aaacctccgg 14940
taggcttttg caaaaagcta attcatccga aaacgttttt cgattaag             14988
```

<210> 3
<211> 353
<212> PRT
<213> Mus musculus

<400> 3

```
Met Gly Cys Cys Leu Ser Glu Glu Ala Lys Glu Ala Arg Arg Ile Asn
1               5                   10                  15

Asp Glu Ile Glu Arg His Val Arg Arg Asp Lys Arg Asp Ala Arg Arg
            20                  25                  30

Glu Leu Lys Leu Leu Leu Leu Gly Thr Gly Glu Ser Gly Lys Ser Thr
        35                  40                  45

Phe Ile Lys Gln Met Arg Ile Ile His Gly Ser Gly Tyr Ser Asp Glu
    50                  55                  60

Asp Lys Arg Gly Phe Thr Lys Leu Val Tyr Gln Asn Ile Phe Thr Ala
65                  70                  75                  80

Met Gln Ala Met Ile Arg Ala Met Asp Thr Leu Lys Ile Pro Tyr Lys
                85                  90                  95

Tyr Glu His Asn Lys Ala His Ala Gln Leu Val Arg Glu Val Asp Val
            100                 105                 110

Glu Lys Val Ser Ala Phe Asp Val Pro Asp Tyr Ala Ala Ile Lys Ser
        115                 120                 125
```

```
Leu Trp Asn Asp Pro Gly Ile Gln Glu Cys Tyr Asp Arg Arg Arg Glu
    130                 135                 140

Tyr Gln Leu Ser Asp Ser Thr Lys Tyr Tyr Leu Asn Asp Leu Asp Arg
145                 150                 155                 160

Val Ala Asp Pro Ala Tyr Leu Pro Thr Gln Gln Asp Val Leu Arg Val
                165                 170                 175

Arg Val Pro Thr Thr Gly Ile Ile Glu Tyr Pro Phe Asp Leu Gln Ser
            180                 185                 190

Val Ile Phe Arg Met Val Asp Val Gly Gly Gln Arg Ser Glu Arg Arg
        195                 200                 205

Lys Trp Ile His Cys Phe Glu Asn Val Thr Ser Ile Met Phe Leu Val
    210                 215                 220

Ala Leu Ser Glu Tyr Asp Gln Val Leu Val Glu Ser Asp Asn Glu Asn
225                 230                 235                 240

Arg Met Glu Glu Ser Lys Ala Leu Phe Arg Thr Ile Ile Thr Tyr Pro
                245                 250                 255

Trp Phe Gln Asn Ser Ser Val Ile Leu Phe Leu Asn Lys Lys Asp Leu
            260                 265                 270

Leu Glu Glu Lys Ile Met Tyr Ser His Leu Val Asp Tyr Phe Pro Glu
        275                 280                 285

Tyr Asp Gly Pro Gln Arg Asp Ala Gln Ala Ala Arg Glu Phe Ile Leu
    290                 295                 300

Lys Met Phe Val Asp Leu Asn Pro Asp Ser Asp Lys Ile Ile Tyr Ser
305                 310                 315                 320

His Phe Thr Cys Ala Thr Asp Thr Glu Asn Ile Arg Phe Val Phe Ala
                325                 330                 335

Ala Val Lys Asp Thr Ile Leu Gln Leu Asn Leu Lys Glu Cys Gly Leu
            340                 345                 350

Phe
```

<210> 4

<211> 1074
<212> DNA
<213> Mus musculus

<400> 4
agatccatgg ggtgcaccct gagcgaggag gccaaggaag cccggaggat caacgacgag 60
atcgagcggc agctgcgcag ggacaagcgc gacgcccgcc gggagctcaa gctgctgctg 120
ctggggacag gggagagtgg caagtcgacc ttcatcaagc agatgaggat catccacggg 180
tcggactact ctgacgaaga caagcgcggc ttcaccaagc tggtgtatca gaacatcttc 240
acggccatgc aggccatgat cagagcgatg gacacactca agatcccata caagtatgaa 300
cacaataagg ctcatgcaca attggttcga gaggttgatg tggagaaggt gtctgctttt 360
gacgtccccg actacgcggc aataaagagc ttgtggaatg atcctggaat ccaggagtgc 420
tacgacagac gacgggaata tcagttatct gactctacca aatactatct gaatgacttg 480
gaccgtgtag ccgacccttc ctatctgcct acacaacaag acgtgcttag agttcgagtc 540
cccactacag ggatcatcga atacccctt gacttacaaa gtgtcatttt cagaatggtc 600
gatgtagggg gccaaaggtc agagagaaga aaatggatac actgctttga aaatgtcacc 660
tccatcatgt ttctagtagc gcttagcgaa tatgatcaag ttcttgtgga gtcagacaat 720
gagaaccgca tggaggagag caaagcactc tttagaacaa ttatcaccta cccctggttc 780
cagaactcct ctgtgattct gttcttaaac aagaaagatc ttctagagga gaaaatcatg 840
tattcccacc tagtcgacta cttcccagaa tatgatggac cccagagaga tgcccaggca 900
gctcgagaat tcatcctgaa aatgttcgtg gacctgaacc ccgacagtga caaaatcatc 960
tactcccact tcacgtgcgc cacagatacc gagaacatcc gcttcgtctt tgcagccgtc 1020
aaggacacca tcctgcagct gaacctgaag gagtgtggcc tcttctgaga taaa 1074

<210> 5
<211> 353
<212> PRT
<213> Mus musculus

<400> 5
Met Gly Cys Thr Leu Ser Glu Glu Ala Lys Glu Ala Arg Arg Ile Asn
1               5                   10                  15

Asp Glu Ile Glu Arg Gln Leu Arg Arg Asp Lys Arg Asp Ala Arg Arg
            20                  25                  30

Glu Leu Lys Leu Leu Leu Leu Gly Thr Gly Glu Ser Gly Lys Ser Thr
        35                  40                  45

Phe Ile Lys Gln Met Arg Ile Ile His Gly Ser Asp Tyr Ser Asp Glu
    50                  55                  60

Asp Lys Arg Gly Phe Thr Lys Leu Val Tyr Gln Asn Ile Phe Thr Ala
65                  70                  75                  80

Met Gln Ala Met Ile Arg Ala Met Asp Thr Leu Lys Ile Pro Tyr Lys
                85                  90                  95

Tyr Glu His Asn Lys Ala His Ala Gln Leu Val Arg Glu Val Asp Val
100 105 110

Glu Lys Val Ser Ala Phe Asp Val Pro Asp Tyr Ala Ala Ile Lys Ser
115 120 125

Leu Trp Asn Asp Pro Gly Ile Gln Glu Cys Tyr Asp Arg Arg Arg Glu
130 135 140

Tyr Gln Leu Ser Asp Ser Thr Lys Tyr Tyr Leu Asn Asp Leu Asp Arg
145 150 155 160

Val Ala Asp Pro Ser Tyr Leu Pro Thr Gln Gln Asp Val Leu Arg Val
165 170 175

Arg Val Pro Thr Thr Gly Ile Ile Glu Tyr Pro Phe Asp Leu Gln Ser
180 185 190

Val Ile Phe Arg Met Val Asp Val Gly Gly Gln Arg Ser Glu Arg Arg
195 200 205

Lys Trp Ile His Cys Phe Glu Asn Val Thr Ser Ile Met Phe Leu Val
210 215 220

Ala Leu Ser Glu Tyr Asp Gln Val Leu Val Glu Ser Asp Asn Glu Asn
225 230 235 240

Arg Met Glu Glu Ser Lys Ala Leu Phe Arg Thr Ile Ile Thr Tyr Pro
245 250 255

Trp Phe Gln Asn Ser Ser Val Ile Leu Phe Leu Asn Lys Lys Asp Leu
260 265 270

Leu Glu Glu Lys Ile Met Tyr Ser His Leu Val Asp Tyr Phe Pro Glu
275 280 285

Tyr Asp Gly Pro Gln Arg Asp Ala Gln Ala Ala Arg Glu Phe Ile Leu
290 295 300

Lys Met Phe Val Asp Leu Asn Pro Asp Ser Asp Lys Ile Ile Tyr Ser
305 310 315 320

His Phe Thr Cys Ala Thr Asp Thr Glu Asn Ile Arg Phe Val Phe Ala
325 330 335

Ala Val Lys Asp Thr Ile Leu Gln Leu Asn Leu Lys Glu Cys Gly Leu
340 345 350

Phe

<210> 6
<211> 12
<212> PRT
<213> Homo sapiens

<400> 6
Arg Pro Arg Leu Ser His Lys Gly Pro Met Pro Phe
1 5 10

<210> 7
<211> 13
<212> PRT
<213> Homo sapiens

<400> 7
Gln Arg Pro Arg Leu Ser His Lys Gly Pro Met Pro Phe
1 5 10

<210> 8
<211> 36
<212> PRT
<213> Homo sapiens

<400> 8
Leu Val Gln Pro Arg Gly Ser Arg Asn Gly Pro Gly Pro Trp Gln Gly
1 5 10 15

Gly Arg Arg Lys Phe Arg Arg Gln Arg Pro Arg Leu Ser His Lys Gly
20 25 30

Pro Met Pro Phe
35

## Description

The present invention refers to a test system for the identification of a ligand for angiotension receptor like-1 (APJ receptor) comprising an APJ receptor or a functional variant thereof and a Gαq or a GαI protein or a hybrid protein which is assembled from portions of different G proteins like Gαq or GαI proteins, a method of screening an APJ receptor ligand using the test system, a method for producing a medicament, a method for diagnosis and prognosis of a heart disease and a kit usable for detection or prognosis of a heart disease.

Within a multicellular organism cells have to communicate with each other to ensure the existence of the whole organism. Due to specialization of each cell, the characteristic function of each cell contributes to the structure and activity of the whole organism. These functions are in general under the control of extracellular signals, which include hormones, neurotransmitters, local hormone etc. Almost all the extracellular signals consist of a change in concentration of a given type of molecule in the environment of the cell. In general the extracellular signal has to be translated into an intracellular signal.

There are different mechanisms by which extracellular signals are received by a cell. Many signal substances are not able to penetrate through the cellular membrane and enter into the cell. Therefore, these extracellular signals have to be transduced into an intracellular signal. A quite successful system for the signal transduction consists of three components, i.e. a G protein coupled receptor, a G protein and an effector protein.

<u>G protein coupled receptors</u>

The family of G protein coupled receptors is a large superfamily of integral membrane proteins. Currently there are about 2,000 G protein coupled receptor sequences available from public databases. Some of the receptors and their endogenous ligands as well as exogenous agonists and antagonists have been known for a long time (e.g. the $\beta_2$-adrenergic receptor). Others are so-called orphan G protein coupled receptors since they do not bind to any known endogenous ligand.

G protein coupled receptors (also known as 7-transmembrane, heptahelical or serpentine receptors) exhibit amino acid sequence as well as structural similarities. They are composed of a single polypeptide chain which spans the cell membrane seven times and consist of an extracellular amino terminus, seven predominantly hydrophobic α-helical-domains (of about 20-30 amino acids) spanning the cell membrane, approximately 20 well-conserved amino acids and a cytoplasmic carboxy terminus.

The binding of an agonist to its receptor mediates a change in receptor conformation whereby the receptor is transferred to its active form. The receptor in its active form activates the G protein and mediates the exchange of GTP for GDP. Antagonists (blockers) stabilise the receptor in its inactive form thus blocking the prevailing downstream signal transduction pathway.

<u>G proteins</u>

G protein coupled receptors transduce the signals via heterotrimeric guanine nucleotide-binding proteins (G proteins). Heterotrimeric G proteins consist of an α (39-42 kDa), a β (35-36 kDa), and a γ subunit (7-10 kDa). The α subunit harbours a binding site for GTP/GDP. G proteins are naturally occurring on the cytoplasmic side of the plasma membrane.

Binding of an extracellular ligand leads to a conformational change in the receptor protein that allows it to make contact with a guanine-nucleotide binding protein (G protein). The activated G protein coupled receptor alters the confirmation of the α subunit and enhances the exchange of GTP for GDP. In the inactive state the βγ dimer is bound to the α subunit. Upon the exchange, the βγ dimer dissociates from the α subunit. Both the activated α subunit and the βγ dimer can influence intracellular effector proteins. The α subunit then catalyses the hydrolysis of GTP into GDP and $P_i$, which causes the inactivation of the α subunit and subsequently the binding of the βγ dimer to the α subunit.

Presently, the G protein α subunit family is grouped in four different classes, i.e. $G_{\alpha s}$, $G_{\alpha i}$, $G_{\alpha q}$ and $G_{\alpha 12}$. $G_{\alpha s}$ and $G_{\alpha i}$ classes stimulate or inhibit adenylyl cyclase activity, respectively. The $G_{\alpha q}$ class stimulates phospholipase C β and includes $G_{\alpha q}$, $G_{\alpha 11}$, $G_{\alpha 14}$ and $G_{\alpha 15/16}$. $G_{\alpha 12}$ is a class similar to, yet distinct from, $G_{\alpha q}$.

In general, G protein coupled receptors activate specifically a particular G protein α subunit family, which leads to the activation or inactivation of a particular signal transduction pathway.

<u>Effector proteins</u>

G protein effect a wide spectrum of biological activities through various intracellular enzymes, ion channels and transporters.

The activated G protein activates or inactivates various signal pathways, thereby activating or inactivating further proteins such as enzymes and ion channels which are called effector proteins. Typical effector proteins are ion channels such as N-type $Ca^{2+}$ channel and G protein coupled inwardly rectifying $K^+$ channel (GIRK) and enzymes such as adenylyl cyclase and phospholipase C.

APJ Receptor

One of the earliest orphan receptors was the APJ receptor (angiotensin receptor like-1), originally identified by O'Dowd et al. (O'Dowd et al., 1993, Gene 136: 355-360). Recently, the endogenous ligand of the APJ receptor, apelin-36, has been identified (Tatemoto et al., 1998, Biochem Biophys Res Commun 251: 471-476). Apelin-36 is a peptide agonist consisting of 36 amino acids, but also shorter variants of apelin-36 (C-terminal peptides of 13-19 amino acids) exhibited agonist properties on the APJ receptor. Apelin is produced through processing from the C-terminal portion of the pre-proprotein consisting of 77 amino acid residues and exists in multiple molecular forms.

Apelin and APJ receptor mRNA have been found to be ubiquitously expressed in peripheral tissue as well as in various regions of central nervous system and also in particular peripheral blood mononuclear cells. In the immune system the APJ receptor is reported to support the entry HIV-1 as a co-receptor with CD-4 (Cayabyab et al., 2000, J Virol 74: 11972-11976). Additionally, it was recently found that the APJ receptor exhibits high level of mRNA expression in the heart (Lee et al., 2000, J Neurochem 74: 34-41). Activity of the endogenous ligand was found in extracts of bovine brain, intestine stomach tissue as well as in the heart.

Apelin has shown to be involved in the suppression of cytokine production from mouse spleen, the promotion of extracellular acidification and inhibition of cAMP production in Chinese hamster ovary cells and in the regulation of blood pressure and blood flow (De Falco at al, 2002, In Vivo 2002 16: 333-336). In the heart it has been shown that apelin induced a dose-dependent positive inotropic effect which seems to be mediated via phospholipase C and protein kinase C (Szokodi et al., 2002, Circ Res 91: 434-440). Furthermore, selective inhibitors of $Na^+/H^+$ exchange isoform-1 (NHE) and reverse mode $Na^+/Ca^{2+}$ exchange (NCX) significantly suppressed response of apelin, indicating their involvement in the signal transduction pathway (Szokodi et al., supra).

Surprisingly, it was now found that cardiovascular diseases are associated with increased expression of the APJ receptor gene resulting in increased level of APJ receptor mRNA as well as APJ receptor protein, which is contrary to the findings in WO 03/013576. Furthermore, apelin expression is enhanced, too. Heart diseases being associated with the increased expression are in particular congestive dilated cardiomyopathy, hypertrophic cardiomyopathy and coronary artery disease.

Coronary arterv disease

Coronary artery disease is the most common form of heart disease. The symptoms include angina pectoris, myocardial infarction, shortness of breath, irregular or faster heart beats etc. It most often results from atherosclerosis, which is caused by plaques made of cholesterol, fatty compounds, calcium, and fibrin. Atherosclerosis of the coronary arteries leads to a reduced lumen of these vessels. As the lumen narrows, resistance to flow increases and myocardial blood flow is compromised and an adequate supply with oxygen is no more guaranteed. In addition to thrombus formation initiated by platelet aggregation, the myocardium may also be injured due to coronary artery disease by one of the other mechanisms. The therapy for coronary artery disease is focused on assuring the sufficient blood flow to the heart and includes medication (β blockers, calcium channel blockers, aspirin, statins and nitrates), angioplasty and bypass surgery.

Cardiomyopathy

This disease of the heart muscle, which is not caused by coronary atherosclerosis, leads to an increased peripheral resistance in lesser or greater circulation. In some instances, heart rhythm is disturbed, leading to irregular heartbeats, or arrhythmias. Usually, the exact cause of the muscle damage is never found, but it is a leading reason for heart transplantation. There are four types of cardiomyopathy:
nonischemic cardiomyopathy, congestive dilated cardiomyopathy, hypertrophic cardiomyopathy and restrictive cardiomyopathy.

Congestive dilated cardiomyopathy

This disease is associated with an enlargement of at least one ventricle which causes a reduction of the heart's pumping ability. As a consequence of this the heart tries to cope with the pumping limitation by further enlarging and stretching (compensation). Patients suffering from congestive dilated cardiomyopathy show in general fatigue, weakness, shortness of breath and swelling of the legs and feet, resulting from fluid accumulation that may also affect the lungs (congestion) and other parts of the body. In most cases a specific cause for the disease is never identified. But some factors such as alcohol, viral infections and particular drugs have been linked to the disease's occurrence. The disease is treated by avoiding complicating factors and controlling the symptoms. Therapy begins with the elimination of obvious risk factors, such as alcohol consumption. Additionally, weight loss and dietary changes may be helpful.

Hypertrophic Cardiomyopathy

In hypertrophic cardiomyopathy, the walls of the left ventricle become thickened and stiff. The greatest thickening tends to occur in septum. The thickening reduces the flow of blood through the heart. Most cases of hypertrophic cardiomyopathy are inherited. In other cases, there is no clear cause. If patients suffering from hypertrophic cardiomyopathy show symptoms (many do not) they suffer from breathlessness and chest discomfort. Other signs are fainting during physical activity, strong rapid heartbeats and fatigue. In advanced stages of the disease, patients may have severe heart failure and its associated symptoms, including fluid accumulation or congestion. Treatments for hypertrophic cardiomyopathy vary but can include reduced physical activity, drugs (p blockers, calcium channel blockers, antiarrhythmic medications and diuretics), pacemakers and surgery.

The fact that APJ receptor and apelin are associated with heart diseases makes them an interesting pharmacological target. This applies particularly with regard to the difficulties in treating heart diseases as detailed above. In many cases a successful and/or long-term therapy or even prophylaxes does not existent.

One object of the present invention was therefore the development of a test system for the identification and characterisation of APJ receptor ligands, which may be used e. g. in the prevention or treatment of heart diseases.

Surprisingly it was found that a test system for the identification of an APJ receptor ligand comprising

(a) an APJ receptor or a functional variant thereof, and

(b) a $G\alpha q$ or a $G\alpha i$ protein or a hybrid protein which is assembled from portions of different G proteins like $G\alpha q$ or $G\alpha i$ proteins ( e.g. a $G_{\alpha\ \Delta 6qi4myr}$ protein),

wherein the binding of said APJ receptor ligand to said APJ receptor or functional variant thereof effects a measurable or detectable signal.

An APJ receptor ligand is any compound which binds specifically to the APJ receptor. A specific binding to the APJ receptor according to the present invention includes, without limitation, binding with a dissociation constant $K_D$ of not exceeding $10^{-4}$ mol/l, preferably not exceeding $10^{-5}$ mol/l. The dissociation constant $K_D$ can determined in competition binding experiments using membranes of human ventricle and $[^{125}I]$-$Pyr^1$)Apelin-13 as radioligand (Katugampola et al, 2001, Br J Pharmacol 132: 1255-1260) according to the following equation:

$$B[L] = [L] / \{[L] + K_{DL}\ (1 + [L^*]/K_{DL^*}\},$$

wherein [L] and [L*] represent the concentration of the APJ receptor ligand and the radioligand, respectively. $K_{DL}$ and $K_{DL^*}$ are the dissociation constants of the APJ receptor ligand and the radioligand, respectively and B[L] is the binding (%) at a particular concentration of the APJ receptor ligand.

In an preferred embodiment of the invention the ligand to be identified is an agonist or an antagonist. Agonists bind to the APJ receptor, induce a conformational change of the same and activate the respective signal transduction pathway which leads to a measurable or detectable signal. Antagonists or blockers also bind to the receptor but do in general not induce signal transduction.

In a more preferred embodiment of the invention the agonist is a full, partial or inverse agonist. Full agonists activate the receptor to a maximal extent. Compounds having a lower effect than a full agonist are called partial agonist, since they stimulate signal transduction but to a lesser extent than a full agonist. An inverse agonist is a ligand which produces an effect opposite to that of the agonist by occupying the same receptor. Especially in systems in which the G protein coupled receptor is over-expressed inverse agonists can be identified. They reduce the basal level of the measurable or detectable signal upon binding to the receptor.

The identified ligands can be used to elucidate the characteristics and function of the APJ receptor. Furthermore, the ligands are potential drugs useful in treatment and prevention of APJ receptor related disorders or disease, preferably heart diseases, most preferably congestive dilated cardiomyopathy, hypertrophic cardiomyopathy and coronary artery disease.

The term "APJ receptor" intends a receptor for apelin. An APJ receptor according to the present invention is any naturally occurring APJ receptor. This includes APJ receptors and variants thereof of different species, preferably vertebrates, more preferably mammals, as well as splice variants orAPJ receptors occurring due to receptor polymorphism as known for other receptors such as $\beta_2$-adrenergic receptor. Preferred mammalian APJ receptors or variants thereof include human (Fig. 2), mouse (Mus musculus, WO 00/68244) and rat (Rattus norvegicus, WO 00/68250) APJ receptor. The human APJ receptor or variants thereof is the most preferred APJ receptor. In any case the receptor must be able of transducing a signal, i.e. modifying a Gαq or a Gαi protein or a hybrid protein which is assembled from portions of different G proteins like Gαq or Gαi proteins upon ligand binding. "Modifying" according to the present invention means activating or inactivating a Gαq or a Gαi protein or a hybrid protein which is assembled from portions of different G proteins like Gαq or Gαi proteins. In general the receptor activates a Gαq or a Gαi protein or a hybrid protein which is assembled from portions of different G proteins like Gαq or Gαi proteins upon agonist binding. In case of an activated receptor, e.g. a constitutive active variant of the APJ receptor, the receptor inactivates a Gαq or a Gαi protein or a hybrid protein which is assembled from portions of different G proteins like Gαq or Gαi proteins upon antagonist binding.

A variant of the APJ receptor according to the present invention is an APJ receptor which binds apelin but does not naturally occur. The binding of apelin to the variant of the APJ receptor can be examined by using the apelin analogue [$^{125}$I]-Pyr$^1$)Apelin-13 as radioligand and performing radioligand binding experiments as described by Katugampola and colleagues (Katugampola et al, 2001, Br J Pharmacol 132: 1255-1260). The term "variant" is used to refer to an oligonucleotide sequence which differs from the naturally occurring APJ receptor sequence in one or more nucleotides. Such a variant oligonucleotide is expressed as a protein variant which, as used herein, indicates a polypeptide sequence that differs from the wild-type polypeptide (APJ receptor) in the substitution, insertion or deletion of one or more amino acids. Preferred are conservative amino acid substitutions, whereby a residue is substituted by another with like characteristics. Typical substitutions are among the aliphatic amino acids, among the amino acids having aliphatic hydroxyl side chain, among the amino acids having acidic residues, among the amide derivatives, among the amino acids with basic residues, or the amino acids having aromatic residues. The variant-polypeptide differs in primary structure (amino acid sequence), but may or may not differ significantly in secondary or tertiary structure or in function relative to the wild-type. The variant can also be a portion of the APJ receptor sufficient for binding of apelin or a receptor chimera including an APJ receptor portion fused to a second G protein coupled receptor portion, wherein the chimera is still able of binding apelin and the second G protein coupled receptor portion is a portion of any other G protein coupled receptor. In any case the receptor must be able of transducing a signal, i.e. modifying a Gαq or a Gαi protein or a hybrid protein which is assembled from portions of different G proteins like Gαq or Gαi proteins (e.g. $G_{\alpha\ \Delta 6qi4myr}$ protein), upon ligand binding.

Three G-proteins have been tested in the APJ assay and could be used for the detection of APJ ligands: $G_{\alpha\ \Delta 6qi4myr}$, $G_{\alpha\ 16}$ , and $G_{\alpha\ i4qi4}$. The $G_{\alpha\ \Delta 6qi4myr}$ protein is (Fig. 1) disclosed in WO 02/04665 ($G_{\alpha\ -6qi4myr}$, SEQ ID NO: 2). This G protein is a hybrid G protein assembled from portions of different G proteins containing additional modifications and combines sequences of various α subunits within one protein. This fusion protein of different G proteins was designed by fusing the $G_\alpha$ receptor recognition region of one G protein to the $G_\alpha$ effector activation region of another G protein. The aim was to provide a G protein hybrid which receives signals from G protein coupled receptors but switches on the signal transduction pathway to which the receptor is naturally not coupled to. This "recoupling" of receptors has the advantage that the assay endpoint (increase in intracellular $Ca^{2+}$ concentration in comparison with adenylyl cyclase inhibition) is more readily accessible through measurement methods and can be used in high throughput screening. To build the $G_{\alpha\ \Delta 6qi4myr}$ protein the 6 highly conserved N-terminal amino acids of the $G_{\alpha q}$ protein were deleted. Additionally, to the lack of the six highly conserved amino acids of the amino terminus the hybrid has a $G_{\alpha i}$ protein sequence at the C terminus. Finally, additional myristoylation/palmitoylation recognition sequences were inserted into the amino-terminal region of the $G_\alpha$ subunit to produce $G_{-6qi4myr}$ protein (i.e. $G_{\alpha\Delta 6qi4myr}$ protein). The protein sequence of -6qi4myr at the amino terminus is MGCC, in contrast to MACC in the original sequence of the -6q variants. The $G_{\alpha\ i4qi4}$ protein is also covered in WO02/04665. This G protein is a hybrid G protein which is assembled from portions of different G proteins containing additional modifications and combines sequences of various Gα protein subunits within one protein. For building up the $G_{\alpha i4qi4}$ protein the six highly conserved amino acids of the $G_{\alpha q}$ protein were deleted. Additionally, to the lack of the six highly conserved amino acids of the amino terminus the first four amino acids (MACC) have been substituted by the first four amino acids of $G_{\alpha i}$ protein (MGCT). Furthermore the $G_{\alpha i4qi4}$ protein has a $G_{\alpha i}$ protein sequence at the C terminus (CGLF). The $G_{\alpha 16}$ protein is disclosed in Offermann et al., (1995) J.Biol.Chem 270, 15175 - 15180 (Ga15 and Ga16 couple a wide variety of receptors to phospholipase C). The measurable or detectable signal is produced by an effector, which is a molecule down-stream from the hybrid G proteins ($G_{\alpha\ \Delta 6qi4myr}$ , $G_{\alpha i4qi4}$ ) and the $G_{\alpha 16}$ protein in the signal transduction pathway. A measurable or detectable signal according to the present invention is any change of concentration of a substance or charging which is mediated upon ligand binding to the APJ receptor. The signal can be for example a concentration change of an intracellular compound, e.g. a second messenger such as diacylglycerol or inositol 1,4,5-trisphosphate or ion such as $Ca^{2+}$. The signal can be measured by any known method using selective electrodes, antibodies, radiolabels, fluorescence markers, enzymes and so on.

In general the hybrid G proteins ($G_{\alpha\ \Delta 6qi4myr}$ , $G_{\alpha i4qi4}$ ) and the $G_{\alpha 16}$ protein couple to various phospholipase C β isoforms, which leads to hydrolysis of membrane-bound phosphatidylinositol 4,5-bisphosphate to give diacylglycerol (DAG) and inositol 1,4,5-trisphosphate ($IP_3$). $IP_3$ releases $Ca^{2+}$ from intracellular depots. Therefore, measurable or detectable signals include changes of DAG, $IP_3$ and $Ca^{2+}$.

Methods for the detection and quantification of DAG are known to the artisan. DAG in crude lipid extracts can be measured by the method of Preiss et al (Preiss et al, 1986, J Biol Chem 261: 8597-8600). Cells are stimulated with a putative APJ agonist and solubilized. Produced DAG is reacted with radiolabelled ATP in the presence of DAG kinase, isolated by chromatography and quantified by counting in a β- or γ-counter. Suitable markers for the ATP include $^{3}H$, $^{32}P$, $^{33}P$, $^{35}S$ (when using e. g. adenosine thiotriphosphate) and $^{14}C$.

Methods for the detection and quantification of $IP_3$ are known to the one of skill in the art. $IP_3$ can be measured using commercial available radioimmoassays (e.g. Amersham, IL, USA) according to the manufacturers' instructions. Another method of measuring $IP_3$ includes the incubation of cells with labelled inositol, stimulation of the cell with a putative APJ receptor agonist, lysis of the cells, anion exchange column separation, and quantification of produced labelled $IP_3$ (Berridge, 1983, Biochem J 212: 849-858). Suitable markers for inositol include $^{3}H$ and $^{14}C$.

In a more preferred embodiment the signal to be measured or detected is a change of $Ca^{2+}$ concentration, most preferably of intracellular $Ca^{2+}$ concentration, which is to be measured or detected easily and which can be utilized in a screening assay with high sample throughput.

Suitable methods for the detection and quantification of $Ca^{2+}$ are known to the artisan (Takahashi et al., 1999, Physiol Rev 79: 1089-1125) and include but are not limited to methods using calcium-sensitive probes. $Ca^{2+}$ is in general measured using fluorescent dyes. There exist several well known protein based and non-protein based $Ca^{2+}$ indicators including aequorin, modified green fluorescent protein-calmodulin chimer, Quin, Indo, Fura, BTC, BAPTA etc. All these methods and fluorescent dyes are well known to the one of ordinary skill in the art.

In general cells are grown and loaded with a fluorescence marker and then stimulated with a putative APJ agonist. Thereafter $Ca^{2+}$ is measured with a fluorescence photometer at particular excitation wavelength and emission wavelengths which depend on the used marker.

In the most preferred embodiment the $Ca^{2+}$ is measured by a FLIPR (Fluorometric Imaging Plate Reader) apparatus (Molecular Devices, CA, USA) which typically measures intracellular $Ca^{2+}$ levels in 96-well and 384-well formats. The assay is performed according to the manufacturer's instructions. This method of measuring $Ca^{2+}$ is highly suitable for high through put assays.

In one embodiment of the invention the test system is located in a genetically engineered cell, into which at least one of the components (a) or (b) (an APJ receptor or a functional variant thereof, a $G_{\alpha\ \Delta 6qi4myr}$ protein) is introduced. The introduced component is referred to as transgene.

These cells include but are not limited to HEK 293, 745-A, A-431, atrial myocytes, BxPC3, C5N, Caco-2, Capan-1, CC531, CFPAC, CHO, CHO K1, COS-1, COS-7, CV-1, EAHY, EAHY 926, F98, GH3, GP&envAM12, H-295 R, H-4-II-E, HACAT, HACAT A131, HEK, HEL, HeLa, Hep G2, High Five, Hs 766T, HT29, HUV-EC R24, HUV-EC-C, IEC 17, IEC 18, Jurkat, K 562, KARPAS-299, L 929, LIN 175, MAt-LYLU, MCF-7, MNEL, MRC-5, MT4, N64, NCTC 2544, NDCK II, Neuro 2A, NIH 3T3, NT2/D1, P19, primary neuronal cells, primary dendritic cells, primary human myoblasts, primary keratinocytes, SF9, SK-UT-1, ST, SW 480, SWU-2 OS, U-373, U-937, and Y-1. Other suitable cells are those known to the one of skill in the art.

Preferred cell lines are HEK 293 cells (primary human embryonal kidney), 3T3 cells (murine embryonal fibroblasts), CHO cells (Chinese hamster ovary), COS-7 cells (African green monkey cell line), HeLa cells (human epithelioid cervical carcinoma), JURKAT cells (human T-cell leukaemia), BHK 21 cell (hamster normal kidney, fibroblast), and MCF-7 cells (human breast cancer).

In another embodiment of the invention the test system is located in a transient or stable transfected cell line or membrane thereof. The procedure for introducing a transgene into a recipient cell is called transfection. Transfection with DNA yields stable as well as unstable (transient) cell lines. Transient cell lines reflect the survival of the transfected DNA in extrachromosomal form; stable cell lines result from the integration into the genome. In case the measuring and detecting of the signal is not limited to whole cells, isolated membranes of such cell lines can be used. Isolated membranes are e.g. provided by harvesting cells, optionally homogenising them and collecting them by centrifugation.

The transgenes can be introduced into the cells by a variety of means known to those knowledgeable_in the art, and adapted to each cell type. Recombinant DNA cloning techniques well known in the art for introducing and expressing a nucleic acid molecule can be used to introduce and express the transgenes. Cells can be transfected using any appropriate means, including viral vectors, chemical transfectants, electroporation, calcium phosphate coprecipitation and direct diffusion of DNA.

As used herein, vectors are agents that transport the transgene into the cell and may include appropriate transcriptional and translational control signals such as a promoter. Vectors can be plasmid, viral or others known in the art. The promoter can be inducible or constitutive, general or cell specific, nuclear or cytoplasmic specific promoter. Selection of promoters, vectors and other elements is a matter of routine design within the level of ordinary skill in the art. Many such elements are described in the literature and are available through commercial suppliers. Usually, the method of transfer includes the transfer of a selectable marker to the cells.

In general a cell line is transfected by any of the means mentioned above wherein the transgene is operatively linked to a selectable marker. Following transfection cells are grown e. g. for some days in enriched media and then switched to selective media. Transfected cells exhibit resistance to the selection and are able to grow, whereas non-transfected cells die in general. Examples for selective markers include puromycin, zeocin, neomycin (neo) and hygromycin B, which confer resistance to puromycin, zeocin, aminoglycoside G-418 and hygromycin, respectively.

Another subject of the invention is a method of screening an APJ receptor ligand, wherein the method comprises the steps of:

(a) providing the test system according to present invention,
(b) providing a test compound,
(c) optionally providing a known APJ receptor ligand, and
(d) measuring or detecting the influence of the test compound on the APJ receptor mediated signal transduction pathway.

The aim of the method of screening is the identification of a test compound as an APJ receptor ligand and its characterisation. The test compound can be any test compound either naturally occurring or chemically synthesized. Naturally occurring test compounds include in particular peptides, preferably peptides showing similarity to the endogenous ligand apelin. This includes apelin fragments as well as peptides having a particular sequence homology (the higher the homology the higher the change of identifying an APJ receptor agonist). But the test compound may also be a non-proteinous ligand. It is a fact that the seven transmembrane domains of the G protein coupled receptors form a binding pocket. It is a matter of common knowledge that non-peptide ligands such as catecholamines (epinephrine, norepinephrine) bind more interiorly in the binding pocket than peptides do (their binding site is more outwards). Consequently a suitable target may also be a smaller (as compared to the peptide ligands) non-peptide compound. It is expected that such smaller compounds exhibit upon binding to the receptor preferably antagonist properties as it is known for the angiotensin receptor (losartan, candesartan).

Depending on the assay design agonists, preferably, full, partial or inverse agonists, and antagonists can be identified.

For the identification of full, partial and inverse agonists the method of screening can be performed carrying out steps (a), (b) and (d). For this the test system is incubated with the test compound in a suitable environment (buffer, temperature, substrate etc.) and for a time sufficient to detect or measure a signal. If the test compound is any kind of agonist the concentration should be high enough to induce a signal. The concentration should not exceed the toxicity level which is in general between 0.1 and 1 mol/l.

If an agonist was identified it can be further characterized by using the test system. Full and partial agonist can be characterized by dose response curves. Hereunto, the test system is incubated with increasing concentrations of the test compound. The signal is plotted against the concentration of the agonist. The resulting curve can be analyzed using the in the following equation:

$$S([C]) = S_{min} + (S_{max} - S_{min}) \,.\, [C]/([C] + EC_{50})$$

[C] = concentration of the test compound C
S([C]) = signal at a particular concentration of test compound C
$S_{min}$ = basal or minimal signal
$S_{max}$ = maximal signal
$EC_{50}$ = concentration of test compound C mediating a half maximal signal

The test compound is then characterized by the $EC_{50}$ and $S_{max}$. If $S_{max}$ is as high as $S_{max}$ induced with a full agonist the test compound is also a full agonist. If it is lower, the test compound is a partial agonist. In general drugs with low $EC_{50}$ values are preferred, since less substance is needed for the same effect as compared to a drug with a higher $EC_{50}$ value.

For the identification of antagonists the method of screening can be performed carrying out steps (a) to (d). Since antagonists in general do not mediate the production of a measurable or detectable signal they cannot be identified directly. Therefore, the binding of an antagonist is identified by blocking the effect of an agonist ((c) known APJ receptor ligand). The test system is incubated with the test compound and an agonist in a suitable environment (buffer, temperature, substrate etc.) and for a time sufficient to detect or measure a signal. If the test compound is any kind of antagonist the concentration should be high enough to inhibit the signal mediated by the agonist. The concentration of the test compound and the agonist should not exceed the toxicity level which is in general between 0.1 and 1 mol/l.

If an antagonist was identified it can be further characterized by using the test system. Hereunto, the test system is incubated with increasing concentrations of the test compound in the presence of a defined concentration of an agonist. The signal is plotted against the concentration of the antagonist. The resulting curve can be analyzed using the in the following equation:

$$S([B]) = S_{min} + (S_{max} - S_{min}) \{1 - [B]/([B] + IC_{50})\}$$

[B] = concentration of the test compound B (blocker = antagonist)
S([B]) = signal at a particular concentration of test compound B
$S_{min}$ = basal or minimal signal
$S_{max}$ = maximal signal
$IC_{50}$ = concentration of test compound B mediating a half maximal inhibition

The blocker constant $K_B$ can be calculated according to:

$$K_B = IC_{50} \{1 + [A]/EC_{50}\}$$

[A] = concentration of the agonist
$EC_{50}$ = concentration of the agonist mediating a half maximal signal

The test compound is then characterized by the $K_B$. In general drugs with low $K_B$-values are preferred, since less substance is needed to block or inhibit the same signal.

In one preferred embodiment of the invention the test compound is provided in the form of a chemical compound library. Chemical compound libraries include are plurality of chemical compounds and have been assembled from any of multiple sources, including chemically synthesized molecules and natural products, or have been generated by combinatorial chemistry techniques. They are especially suitable for high throughput screening. They may be comprised of chemical compounds of a particular structure or compounds of a particular creature such as a plant. In the context with the present invention the chemical compound library is preferably a library comprising proteins and polypeptides or ligands for G protein coupled receptors.

In one preferred another embodiment of the invention the method of screening a ligand is carried out using whole cells. The cells can be any known in the art. Examples of useful cells are mentioned above. Usually cells are grown in multiwell plates until use. Cells are optionally labelled (if necessary) and subsequently stimulated with an APL receptor ligand. Using whole cells is advantageous in contrary to membranes, since intracellular substrates, enzymes etc. need not to be added to the test system. Furthermore, whole cells in multiwell plates are particularly suitable for high trough put screening test and automated test systems.

Advantageously the method of the present invention is carried out in a robotics system e.g. including robotic plating and a robotic liquid transfer system, e.g. using microfluidics, i.e. channelled structured.

In another embodiment of the present invention, the method is carried out in form of a high-through put screening system. In such a system advantageously the screening method is automated and miniaturized; in particular it uses miniaturized wells and microfluidics controlled by a roboter.

Another subject of the present invention is a method for producing a medicament, wherein the method comprises the steps of:

(a) identifying an APJ receptor ligand carrying out the method of screening according to the present invention,
(b) providing adequate amounts of the ligand, and
(c) formulating the measured or detected test compound with one or more pharmaceutically acceptable carriers or auxiliary substances.

The medicament is preferably used for the prophylaxis, therapy or diagnosis of an APJ-receptor related disease or disorder. An APJ receptor-related disease or disorder is a condition characterized in that the expression level of the receptor, of its endogenous ligand, its the signal transduction, its signal transduction pathway or the effects of an down-stream effector molecule is changed. Alternatively, the APJ receptor is involved in the development or progression of a disease or disorder. Examples of such diseases include, but are not limited to, cardiovascular diseases, heart diseases; malfunction of blood pressure regulation, diseases of the nervous system and the immune system, such as HIV.

More preferably, the disease is a heart disease, most preferably congestive dilated cardiomyopathy, hypertrophic cardiomyopathy and coronary artery disease.

For the medication the isolated test compound or its pharmaceutically acceptable salt has to be in a pharmaceutical dosage form in general consisting of a mixture of ingredients such as pharmaceutically acceptable carriers or auxiliary substances combined to provide desirable characteristics.

The formulation comprises at least one suitable pharmaceutically acceptable carrier or auxiliary substance. Examples of such substances are demineralised water, isotonic saline, Ringer's solution, buffers, organic or inorganic acids and bases as well as their salts, sodium chloride, sodium hydrogencarbonate, sodium citrate or dicalcium phosphate, glycols, such a propylene glycol, esters such as ethyl oleate and ethyl laurate, sugars such as glucose, sucrose and lactose, starches such as corn starch and potato starch, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils such as groundnut oil, cottonseed oil, corn oil, soybean oil, caster oil, synthetic fatty acid esters such as ethyl oleate, isopropyl myristate, polymeric adjuvans such as gelatin, dextran, cellulose and its derivatives, albumins, organic solvents, complexing agents such as citrates and urea, stabilizers, such as protease or nuclease inhibitors, preferably aprotinin, ε-aminocaproic acid or pepstatin A, preservatives such as benzyl alcohol, oxidation inhibitors such as sodium sulphite, waxes and stabilizers such as EDTA. Colouring agents, releasing agents, coating agents, sweetening, flavouring and perfuming agents, preservatives and antioxidants can also be present in the composition. The physiological buffer solution preferably has a pH of approx. 6.0-8.0, especially a pH of approx. 6.8-7.8, in particular a pH of approx. 7.4, and/or an osmolarity of approx. 200-400 milliosmol/liter, preferably of approx. 290-310 milliosmol/liter. The pH of the medicament is in general adjusted using a suitable organic or inorganic buffer, such as, for example, preferably using a phosphate buffer, tris buffer (tris(hydroxymethyl)aminomethane), HEPES buffer ([4-(2-hydroxyethyl)piperazino]ethanesulphonic acid) or MOPS buffer (3-morpholino-1-propanesulphonic acid). The choice of the respective buffer in general depends on the desired buffer molarity. Phosphate buffer is suitable, for example, for injection and infusion solutions. Methods for formulating a medicaments as well as suitable pharmaceutically acceptable carrier or auxiliary substance are well known to the one of skill in the art. Pharmaceutically acceptable carriers and auxiliary substances are a. o. chosen according to the prevailing dosage form and identified compound.

The pharmaceutical composition can be manufactured for oral, nasal, rectal, parenteral, vaginal, topic or vaginal administration. Parental administration includes subcutaneous, intracutaneous, intramuscular, intravenous or intraperitoneal administration.

The medicament can be formulated as various dosage forms including solid dosage forms for oral administration such as capsules, tablets, pills, powders and granules, liquid dosage forms for oral administration such as pharmaceutically acceptable emulsions, microemulsions, solutions, suspensions, syrups and elixirs, injectable preparations, for example, sterile injectable aqueous or oleaginous suspensions, compositions for rectal or vaginal administration, preferably suppositories, and dosage forms for topical or transdermal administration such as ointments, pastes, creams, lotions, gels, powders, solutions, sprays, inhalants or patches.

The specific therapeutically effective dose level for any particular patient will depend upon a variety of factors including the activity of the identified compound, the dosage form, the age, body weight and sex of the patient, the duration of the treatment and like factors well known in the medical arts.

The total daily dose of the compounds of this invention administered to a human or other mammal in single or in divided doses can be in amounts, for example, from about 0.01 to about 50 mg/kg body weight or more preferably from about 0.1 to about 25 mg/kg body weight. Single dose compositions may contain such amounts or submultiples thereof to make up the daily dose. In general, treatment regimens according to the present invention comprise administration to a patient in need of such treatment from about 10 mg to about 1000 mg of the compound(s) of the compounds of the present invention per day in single or multiple doses.

Still another subject of the present invention is a method for diagnosis or prognosis for a heart disease comprising the following steps:

(a) determining the expression level of APJ receptor gene and/or apelin gene in a sample to be tested,
(b) comparing the expression level of APJ receptor gene and/or apelin gene in the sample to be tested determined in step (a) with the expression level of APJ receptor gene and/or apelin gene in a control probe, and
(c) identifying a sample with increased APJ receptor and/or apelin gene expression.

The APJ receptor and apelin can be used as indices of a heart disease, preferably congestive dilated cardiomyopathy, hypertrophic cardiomyopathy and coronary artery disease. Surprisingly the inventors found that the expression of APJ receptor as well as apelin was increased in diseased heart (table 1). Therefore, an increased expression of APJ receptor and apelin is obviously an indicator for heart diseases such as congestive dilated cardiomyopathy, hypertrophic cardiomyopathy and coronary artery disease.

In the method for diagnosis or prognosis according to the present invention, expression of the APJ receptor gene or the apelin gene can be assayed by a nucleic acid amplification method, hybridization method, enzyme immunoassay, fluorescence immunoassay, luminescent immunoassay or protein bioassay which include membrane, solution, or chip-based technologies for the detection and/or quantification of nucleic acid or protein and so on.

In a preferred embodiment the method for diagnosis and prognosis is carried out by determining the expression levels of the gene(s) on the basis of levels of mRNAs transcribed from the gene(s) or levels of polypeptides translated from the genes.

A wide variety of labels and conjugation techniques are known by those skilled in the art and can be used in various nucleic acid and amino acid assays. Means for producing labelled hybridization or PCR probes for detecting sequences related to polynucleotides encoding APJ receptor or apelin include oligolabelling, nick translation, end-labelling, or PCR amplification using a labelled nucleotide. Alternatively, sequences encoding APJ receptor or apelin can be cloned into a vector for the production of an mRNA probe. Such vectors are known in the art, are commercially available, and can be used to synthesize RNA probes in vitro by addition of labelled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

A variety of techniques known in the art can be used to quantify the level at which a given polypeptide or protein is expressed. These include, but are not limited to immunological techniques such as an ELISA, RIA, or western blot, or quantitative analytical techniques such as spectroscopy or flame chromatography.

In a more preferred embodiment the method for diagnosis and prognosis is carried out by determining the levels of mRNAs by a hybridization method or a nucleic acid amplification method. Such methods are known to the artisan and include the dot blot hybridization method, the Northern hybridization method or the RT-PCR method.

In another more preferred embodiment the method for diagnosis and prognosis is carried out by determining the levels of polypeptides by using binding proteins, e.g. antibodies or anticalins, capable of binding specifically to the polypeptides or fragments thereof.

A variety of protocols for detecting and measuring polypeptides using either polyclonal or monoclonal antibodies specific for the polypeptide, are known in the art. Examples include enzyme-linked immunosorbent assay (ELISA), radioimmunoassay (RIA), and fluorescence activated cell sorting (FACS).

For this method the antibodies capable of binding specifically to polypeptides encoded by the APJ receptor gene and/or apelin gene for carrying out the prevailing test (see above) are used. The procedure for preparing an antibody or antibody fragment is effected in accordance with methods which are well known to the skilled person, e. g. by immunizing a mammal, for example a rabbit, with the APJ receptor or apelin, where appropriate in the presence of, for example, Freund's adjuvant and/or aluminium hydroxide gels (see, for example, Diamond, B.A. et al. (1981) The New England Journal of Medicine: 1344-1349). The polyclonal antibodies which are formed in the animal as a result of an immunological reaction can subsequently be isolated from the blood using well known methods and, for example, purified by means of column chromatography. Monoclonal antibodies can, for example, be prepared in accordance with the known method of Winter & Milstein (Winter, G. & Milstein, C. (1991) Nature, 349, 293-299).

Another subject of the present invention is a kit usable for detection or prognosis of a heart disease, comprising primers and/or a probe which is usable for determining expression levels of the APJ receptor gene and/or apelin gene.

The kit usable for detection and prognosis of a heart disease includes a kit for examining alterations in the expression levels of the APJ receptor and/or apelin gene in a sample to be tested. The kit of the present invention is a kit capable of quantifying the level of mRNA transcribed from the above-mentioned genes. In order to examine expression the kit comprises primers and/or a probe which is usable for determining the level of mRNA from the gene.

The kit of the present invention is based on the surprising findings that APJ receptor and apelin gene expression is increased in the hearts of patients with heart disease, in particular congestive dilated cardiomyopathy, hypertrophic cardiomyopathy and coronary artery disease.

The primers are designed to specifically bind to nucleic acids corresponding to the above-mentioned genes. They include any primers which are capable of specifically amplifying nucleic acid sequences derived from mRNA transcribed from the above-mentioned genes under reaction conditions used for any conventional nucleic acid amplification method, e.g. RT-PCR. For instance, primers can be designed and synthesized on the basis of the nucleotide sequences of these genes. In an preferred embodiment the kit comprises a pair of primers capable of specifically amplifying a nucleic acid sequence derived from mRNA transcribed from the above-mentioned genes. Furthermore the kit may comprise suitable reagents for carrying out the amplification the mRNA and quantification of the gene expression.

Another subject of the present invention is a kit usable for detection or prognosis of a heart disease, comprising antibodies capable of binding specifically to polypeptides encoded by the APJ receptor gene and/or apelin gene.

A variety of techniques known in the art can be used to quantify the level at which a given protein is expressed. For further details see above.

Table 1:

| Comparison of Gene Expression in Diseased and Normal Nonfailing Heart | | |
|---|---|---|
| | Gene expression (% of total samples) | |
| | APJ receptor | Apelin |
| Normal nonfailing heart (n = 52) | 26* [70] | 15* [30] |
| Diseased heart (n = 462)<br>• *Congestive* dilated cardiomyopathy (n = 124)<br>• *Coronary artery disease (n = 301)*<br>• *Hypertrophic cardiomyopathy (n = 37)* | 92* [465]<br>95* *[465]*<br>*92* [410]*<br>*83* [506]* | 64* [61]<br>62* *[55]*<br>*63* [63]*<br>*72* [66]* |

* Indicates the % of samples in given sample set wherein the gene was determined to be expressed. The median expression value (in arbitrary units) for sample set is given in [ ].

Description of the Figures

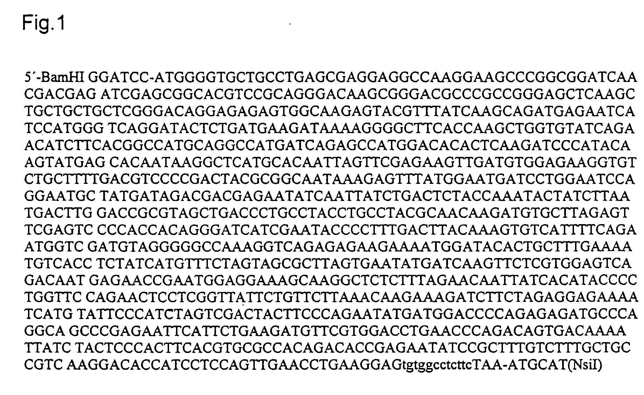

Fig. 1 depicts the polynucleotide sequence of the mouse $G_{\alpha\ \Delta 6qi4myr}$ protein coding region inserted into pCDNA 1 (corresponding to Seq ID No. 1)
The $G_{\alpha\ \Delta 6qi4myr}$ protein gene was inserted into pCDNA 1 5' and 3' via a BamHI and a NsiI site, respectively.

Fig. 2 shows the human apelin receptor expression plasmid and the amino acid sequence of the receptor protein (corresponding to Seq ID No. 2)
The human apelin receptor gene was cut from AVEM1139400 and inserted into pEAK8 which was cut with EcoRI.

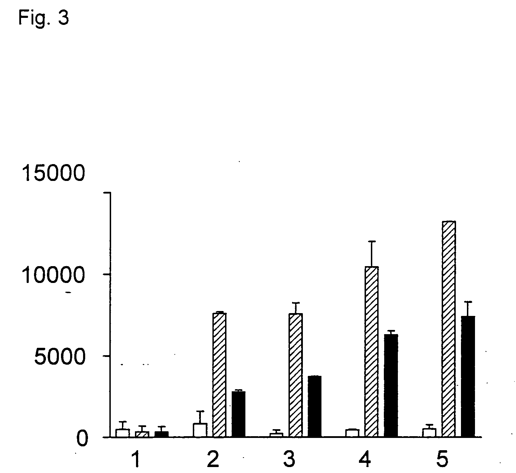

Fig. 3 shows the effects of various apelin peptides on the APJ receptor in transiently cotransfected CHO cells
The APJ receptor was transiently cotransfected in CHO cells (Chinese hamster ovary cells) with vector DNA as a control (white column) or the G protein α subunits α16 (black column) and α Δ6qi4myr (striped column) according to methods known to the one skilled in the art. Cells were challenged with 1 μmol/l of peptide each:

1 apelin like peptide/jerini peptide
2 apelin 12
3 apelin 13
4 apelin 36
5 pyr-apelin

and the intracellular $Ca^{2+}$ concentration determined in a functional FLIPR calcium assay. Peptides 2-5 induced intracellular calcium release via the APJ receptor. It was shown that functional calcium responses required the presence of a chimeric/promiscuous G protein α subunit. Data presented are mean values (fluorescence intensity units, FIU) from 2 individual experiments performed in duplicate.

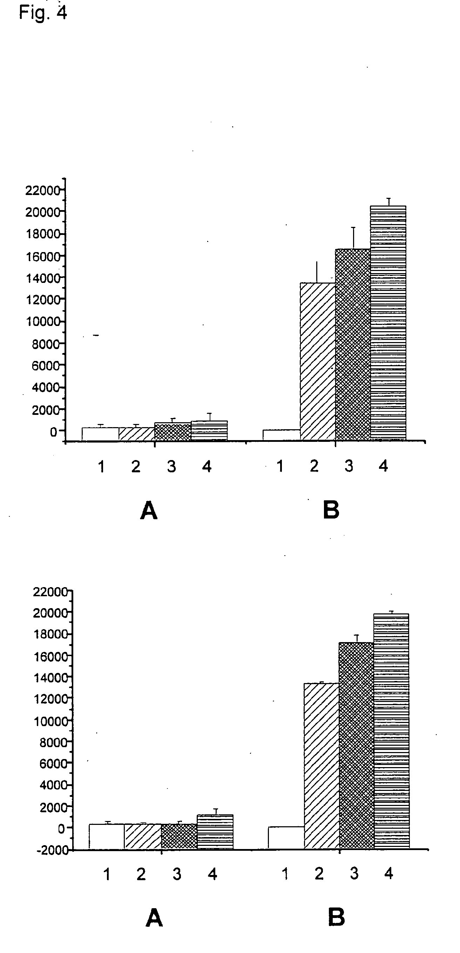

Fig. 4 shows dose-dependently the effects of apelin on the APJ receptor in stably transfected HEK 293 cells overexpressing $G_{\alpha i4qi4}$ and APJ receptor
HEK 293 cells were stably transfected with $G_{\alpha\ \Delta 6qi4myr}$ alone (A) or cotransfected with $G_{\alpha\ \Delta 6qi4myr}$ and APJ (B) as described above and incubated with

1 none
2 50 nmol/l
3 200 nmol/l
4 1 μmol/l

pyr-apelin-13 (top) or apelin-36 (bottom). Both peptides induced intracellular calcium release via the APJ receptor in a dose-dependent manner. It was shown that functional calcium responses required the presence of the APJ receptor. Data presented are mean values (fluorescence intensity units, FIU) from 2 individual experiments performed in duplicate.

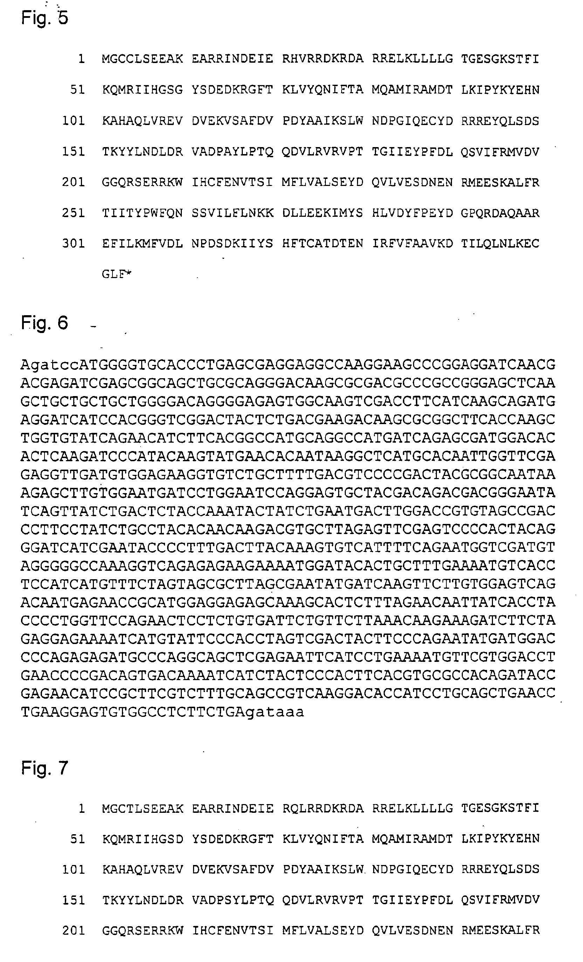

Fig. 5 depicts the amino acid sequence of $G_{\alpha\Delta 6qi4myr}$ protein ( corresponding to Seq ID No. 3)

Fig. 6 depicts the polynucleotide sequence of the $G_{\alpha i4qi4}$ gene coding region (corresponding to Seq ID No. 4)

Fig. 7 depicts the amino acid sequence of $G_{\alpha i4qi4}$ protein (corresponding to Seq ID. No. 5)

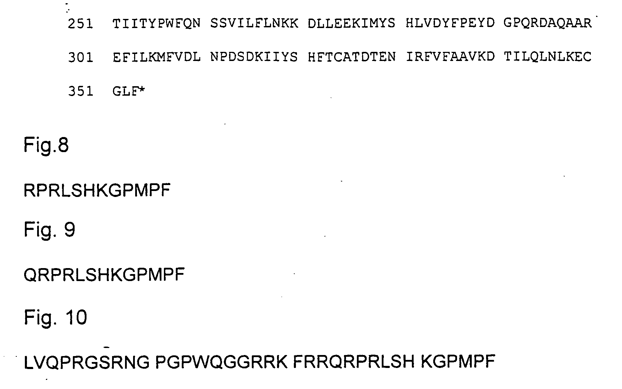

Fig. 8 depicts the amino acid sequence of human apelin-12 (corresponding to Seq ID No. 6)

Fig. 9 depicts the amino acid sequence of human apelin-13 (corresponding to Seq ID No. 7) .

Fig. 10 depicts the amino acid sequence of apelin-36 (corresponding to Seq ID No. 8).

Description of Seq IDs:

Seq ID No. 1: Polynucleotide sequence of mouse $G_{\alpha\Delta 6qi4myr}$ protein coding region inserted into pCDNA 1

Seq ID No. 2: Polynucleotide sequence of human apelin receptor expression plasmid (human apelin receptor gene was cut from AVEM1139400 and inserted into pEAK8 which was cut with EcoRl) and the amino acid sequence of the receptor protein.

Seq ID No. 3: Amino acid sequence of mouse $G_{\alpha\ \Delta 6qi4myr}$ protein.

Seq ID No.4: Polynucleotide sequence of mouse $G_{\alpha i4qi4}$ gene coding region.

Seq ID No. 5: Amino acid sequence of mouse $G_{\alpha i4qi4}$ protein.

Seq ID No. 6: Amino acid sequence of human apelin-12.

Seq ID No. 7: Amino acid sequence of human apelin-13.

Seq ID No. 8: Amino acid sequence of human apelin-36.

Examples

1. <u>Functional APJ receptor/$G_{\alpha i4qi4}$ protein cell-line</u>

1.1. Cell culture

HEK 293 cells (human embryo kidney cells) were grown in DMEM (Dulbecco's modified Eagle's medium) with 10% FCS (fetal calf serum) at 37°C (5% $CO_2$). After the second transfection the antibiotics zeocin and puromycin were added to the cells. Cells were passaged at two- or three-day intervals.

1.2. Transfection of a HEK293 cell line with the $G_{\alpha i4qi4}$ Protein

For transfection cells were seeded in 100-mm plates. About 24 hours later, the cells were transfected withthe pHOOK3 vector (Invitrogen, MD, USA) carrying the $G_{\alpha i4qi4}$ (Fig. 6). Stabile cell lines were stored frozen until use.

1.3. Transfection of the cell line with the APJ receptor

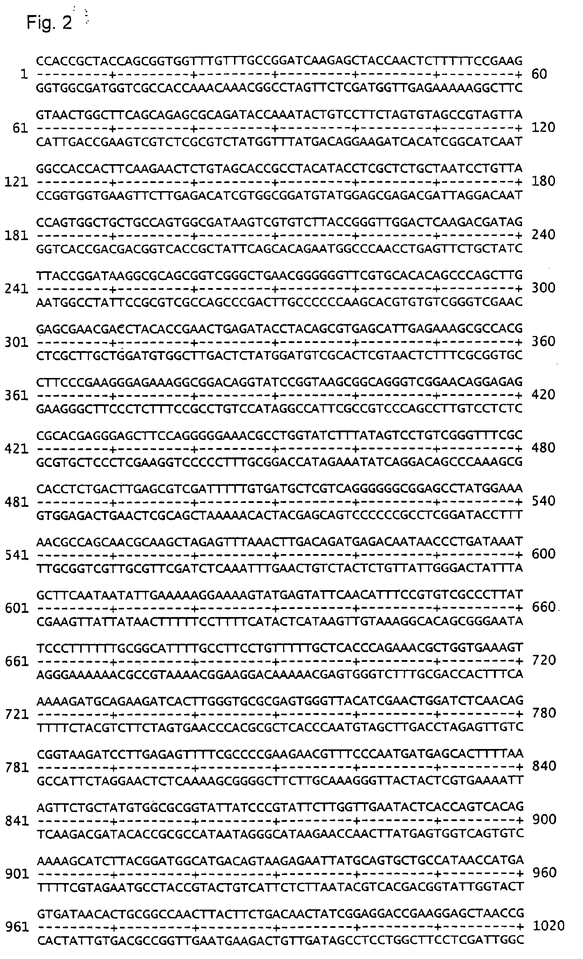

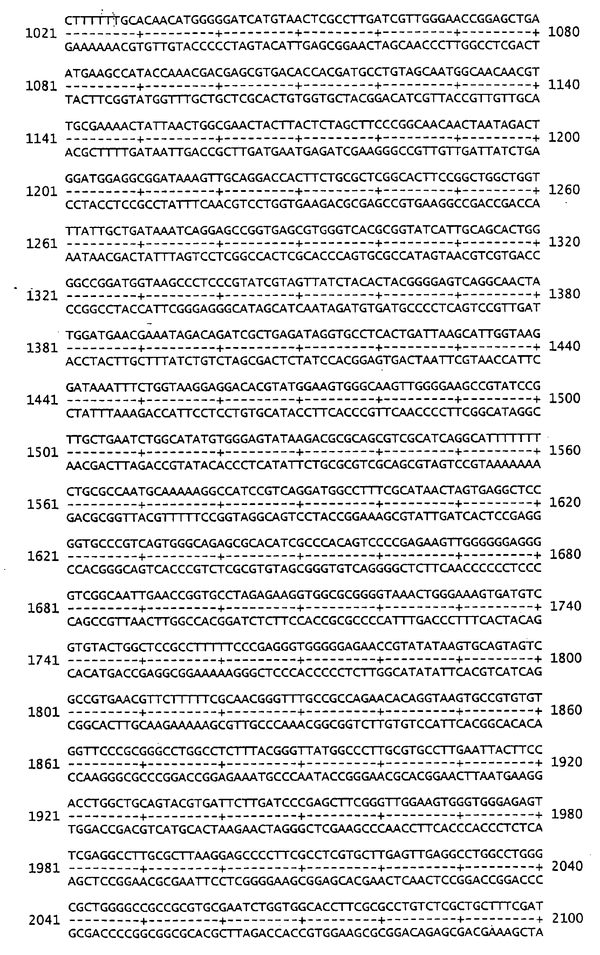

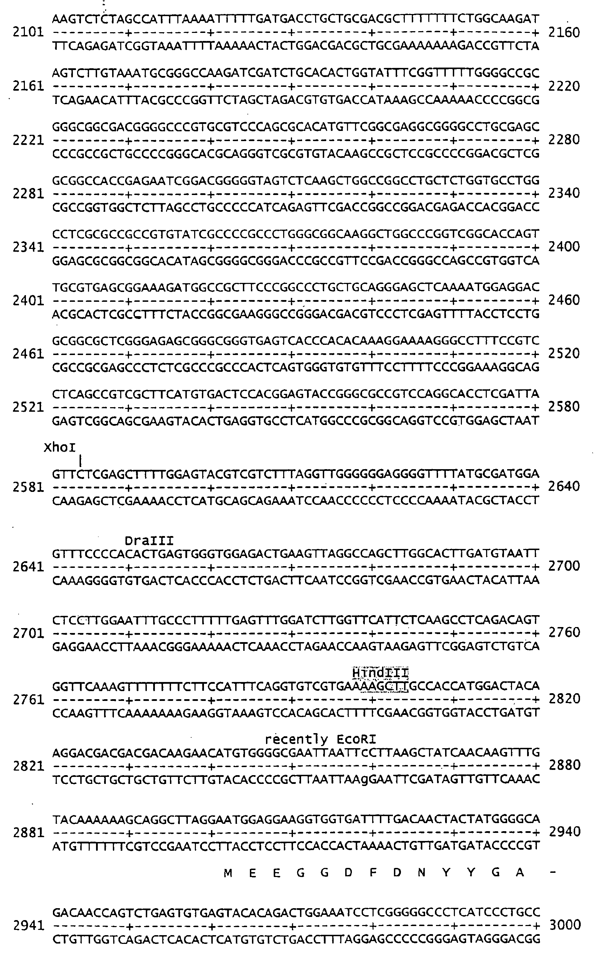

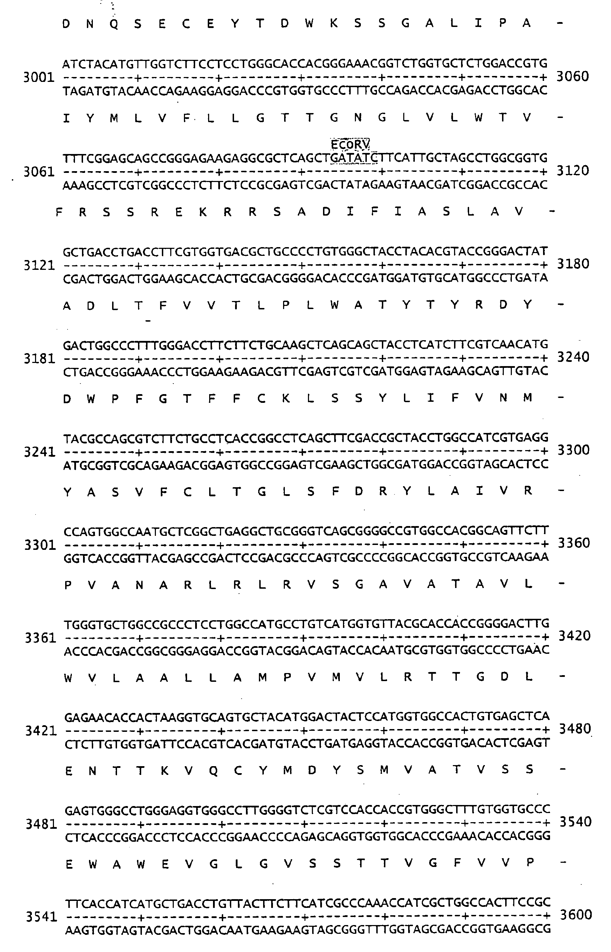

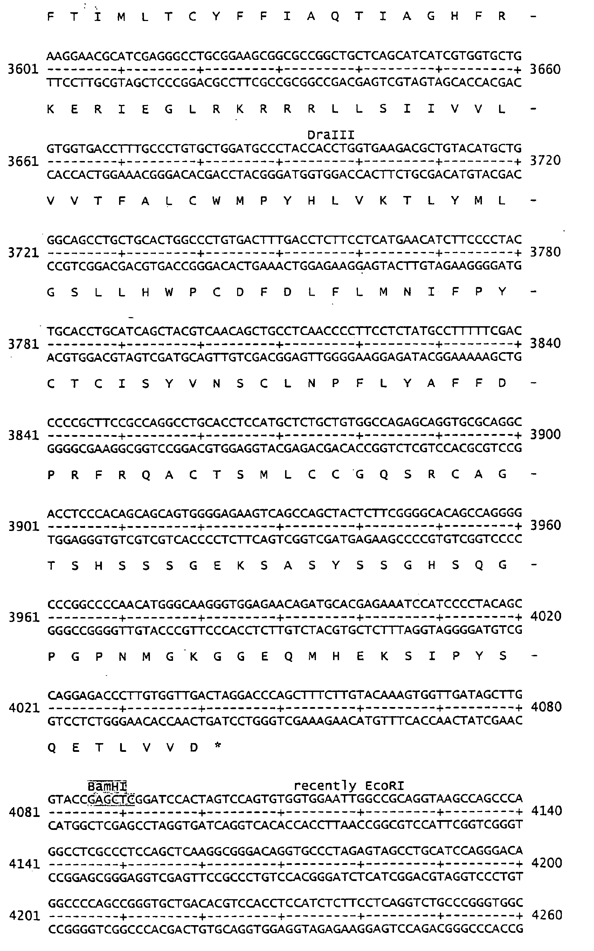

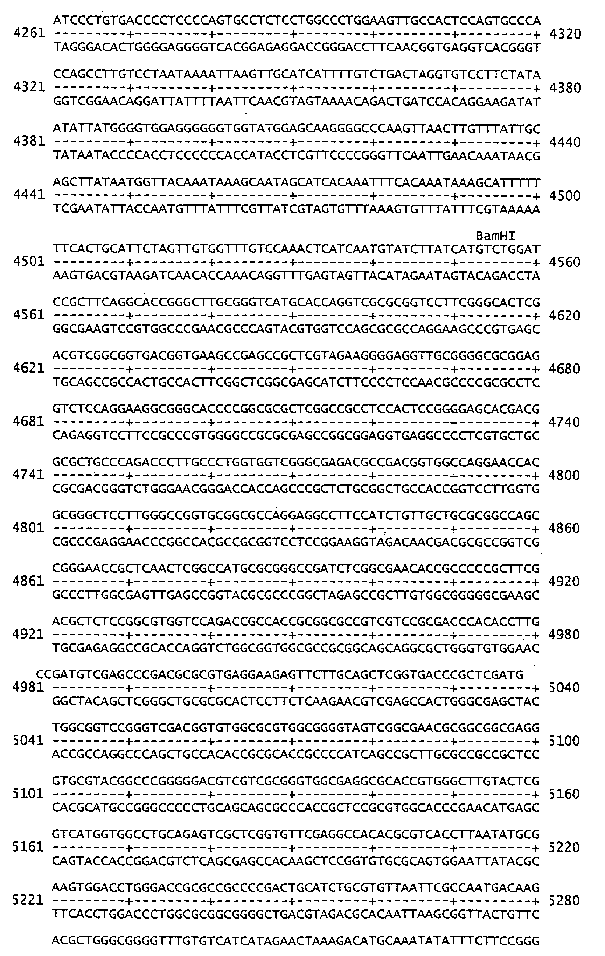

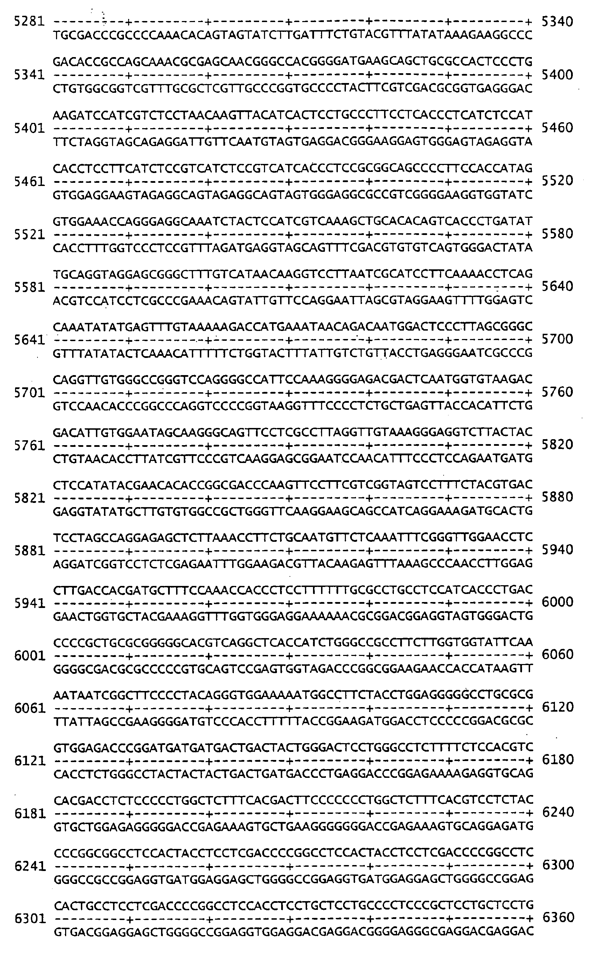

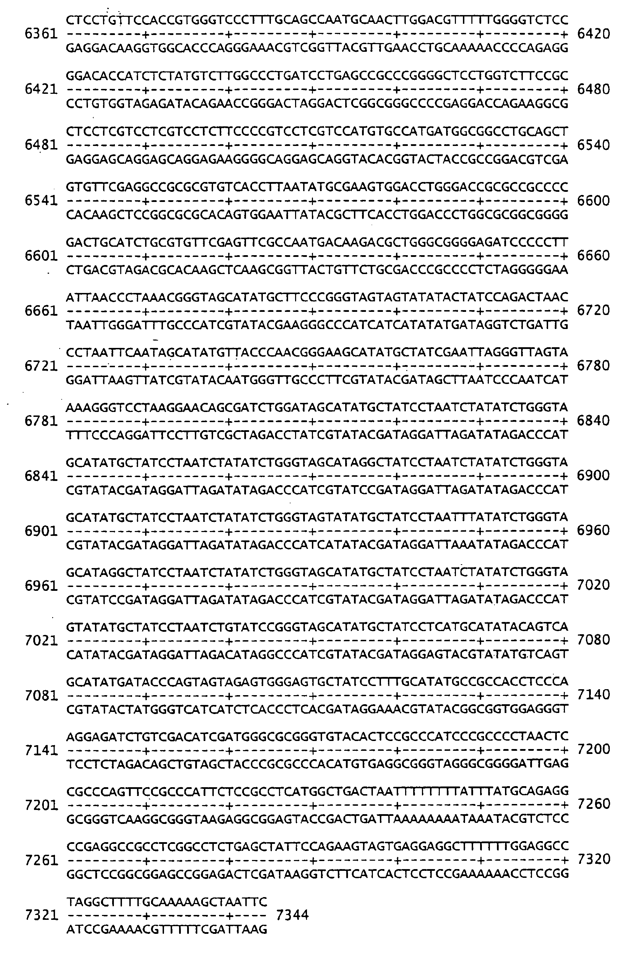

Stably transfected HEK 293 cell line (overexpressing $G_{\alpha i4qi4}$ gene) was grown on 100-mm plates with 10 ml DMEM (10 % FCS). After 24 hours, medium was removed and 8 ml fresh DMEM (10 % FCS) was added. 12 µl FuGENE 6 transfection reagent (Roche, Germany) and 400 µl OptiMEM was mixed and incubated for 5 min. Thereafter 4 µg endotoxin-free plasmid DNA (APJ/pEAK8; see Fig. 2) was added and samples were incubated for another 10 min. The transfection mixture was added to the cells. After 24 hours medium was exchanged with fresh medium containing zeocin (250 µg/ml) and puromycin (500 ng/ml). To select stable cell lines cells were cultivated for 6 weeks prior to use in functional tests. Cells without antibiotic resistance died after approximately 2 weeks.

2. FLIPR Calcium Assay

The FLIPR calcium assay provides a suitable method for detecting changes in intracellular calcium concentration. It is a fluorescence-based assay which was performed according to the manufacture's instructions (Molecular Devices, CA, USA). Briefly summarized, cells were seeded on 96- or 384-well plates Loading Buffer was added to the cells and plates incubated for 1 hour at 37°C. After incubation cells were stimulated with the prevailing APJ receptor ligand or none.

Thereafter intracellular calcium concentration was measured in the FLIPR according to the FLIPR system manual.

2.1 Chemicals and Equipment

| | |
|---|---|
| Materials for cell culture | |
| DMEM | Gibco 41965-039 |
| FCS gold | PAA A15-649, Chg.: A01121-170 |
| NON-ESSENTIAL AMINO ACIDS (NEAs) | Gibco 11140-035 |
| Zeocin | I nvitrogen 45-0003 |
| Puromycin x 2 HCl | MoBiTec 80018 |
| TRYPSIN/EDTA solution | Biochrom AG L2143 |
| PBS w/o Ca and Mg | GIBCO 14190-094 |
| | |
| Materials for FLIPR assay | |
| Probenicid | Sigma P-8761 |
| Pluronic F127 | Molecular probes P-3000 |
| Fluo-4/AM | Molecular probes F-14202 |
| | |
| Apelin 12 | Phoenix 057-23 |
| Apelin 13 | Bachem H-4566 |
| Apelin 36 | Bachem H-4896 |
| Pyr-Apelin 13 | Bachem H-4568 |

| Cell culture medium | 500 ml DMEM | Final concentration |
|---|---|---|
| | 50 ml FCS | 10% |
| | 5.5 ml NEAs | 1 x |
| | 1.389 ml Zeocin | 250 µg/ml |
| | 0.278 ml Puromycin | 500 ng/ml |
| Assay and dilution buffer | 1.5ml 1 M $CaCl_2$ | 1.5 mM |
| | 27.0 ml 5 M NaCl | 135 mM |

(continued)

| | | |
|---|---|---|
| | 5.0ml 1 M KCl | 5 mM |
| | 1.0 ml 1 M MgS04 | 1 mM |
| | 20.0 ml 1 M HEPES pH7.4 | 20 mM |
| | 5.0 ml 1 M Glucose | 5 mM |
| | 10.0 ml 250 mM Probenicid in 1 N NaOH | 2.5 mM |
| | | |
| | ad 1 l , pH7.4 with HCl conc. | |
| | | |
| Dye loading buffer | Mix: | |
| | 24 µl Pluronic F127 | 0.046 % |
| | + 24 µl Fluo-4, AM | |
| | Add to | |
| | 10.5 ml assay buffer | |
| | | |
| Peptide solutions (store at +4°C) | All peptides were solved in water ad | Molecular weight/peptide content |
| Apelin 12 | 140.57 µM | 1422.73 |
| Apelin 13 | 493.27 µM | 1550.86 g/mol / 76.5% |
| Apelin 36 | 173.5 µM | 4195.9 g/mol / 72.8 % |
| Pyr-Apelin 13 | 466.6 µM | 1534.8 g/mol / 71.6% |

| Equipment | | |
|---|---|---|
| Tissue Culture Flasks, 75cm$^2$ Filter cap | Greiner | 658 175 |
| 96 Well Black/ Clear Plate Poly-D-Lysine Cellware | Becton Dickinson | 35 4640 |
| PS Microplate 96 well, flat bottom | Greiner | 655101 |
| PS Cover plate | Greiner | 656101 |
| Multi channel pipette (8 well, 12 well) | Micronic | |
| 22-Coulter counter | Beckman | 9914558 |
| Cell washer Columbus plus | Tecan | F 109204 |
| | | |
| FLIPR | Molecular Dynamics | |

2.2 Cell line: HEK293-PSCi4qi4-APJ

HEK293-PSCi4qi4 cells were stably transfected with pEAK8-APJ. Cells are grown in DMEM medium with 10% FCS supplemented with zeocin (250 µg/ml) and puromycin (500 ng/ml).

2.3 FLIPR assay procedure (established for 96-well format)

For measuring calcium transients cells are grown in flat-bottom, black-wall 96-well poly-D-lysine plates. Cells are seeded at a density of 60,000 cells/well in 200 µl and cultured over night (ca. 18 h).

For dye-loading the culture medium is carefully removed and replaced by 100 µl/well dye loading buffer. Cells are incubated for 1 h at 37°C, and then washed 3x with assay buffer. After washing 90 µl buffer/well are left.

Apelin peptides were added in 90 µl assay buffer (2x concentrated).

| | |
|---|---|
| Parameters of measurement: | Sample interval: 2 sec |
| | Sample count: 60 |
| | Fluid volume: 90µl |
| After sample: 6 | Pipette height: 130 µl |
| | Dispense speed: 45 µl/sec |
| | Mix after addition: yes |

2.4 Determination of EC50 of apelin peptides

EC50 values of apelin peptides were determined in two independent measurements:

| EC50 | nM | nM |
|---|---|---|
| Apelin-12 | 57 | 61 |
| Apelin-13 | 29 | 37 |
| Apelin-36 | 51 | 27 |
| Pyr-apelin-13 | 34 | 34 |

2.5 Determination of Z' factor

The Z' factor is defined by

$$Z' = 1 - \frac{3 * \text{SD high control} + 3 * \text{SD low control}}{\text{mean high control - mean low control}}$$

and is used as a measure to judge the suitability of an assay for high throughput screening. Interpretation of the Z' factor is aided by a classification scheme as shown.

Z' factor was determined in two independent measurements using 500 nM apelin-13 in one half and assay buffer in the other half of a 96-well plate. Calculated mean Z' factor was 0.91.

( Classification:

| | |
|---|---|
| ideal assay | $z = 1$ |
| excellent assay | $1 > z \geq 0.5$ |
| ± acceptable | $0.5 > z > 0$ |
| ' yes/no ' type : | $z = 0$ |
| screening impossible | $z < 0$ ) |

2.6 Determination of DMSO dependency

EC50 values of apelin-13 were determined in presence of increasing concentrations of DMSO (0-10%). A maximum final concentration of 2% DMSO in the assay is tolerated without influencing the EC50 value of apelin-13.

2.7 Determination of stability of apelin peptides

According to the manufacturer's instructions apelin peptides were solved in sterile water and stored at +4°C. Thus, apelin peptides are stable and give reproducible EC50 values. When left at room temperature for 5 hours (in the final concentration in assay buffer), EC50 values of apelin peptides drop by a factor of at least 2-fold indicating that peptides are not stable under these conditions.

3. Gene Expression Levels of APJ Receptor and apelin in normal and diseased heart

A gene expression library providing expression profiles for a total number of 514 well defined normal and diseased heart samples, accompanied by clinical patient information was generated according to standard methods known to the skilled in the art. Gene expression in normal and diseased heart was compared using HG-U95A-E Gene-Chips (Affymetrix, Santa Clara, CA). The APJ receptor gene was upregulated in diseased heart more than sixfold (Table 1). More detailed analysis indicated that APJ receptor is significantly upregulated in heart samples from patients with congestive dilated cardiomyopathy (n = 124), coronary artery disease (n = 301) and hypertrophic cardiomyopathy (n = 37) when compared to normal nonfailing heart as control (n = 52). A similar expression pattern could be obtained from apelin, the endogenous peptide ligand for APJ receptor, where the gene was upregulated in diseased heart more than twofold. No significant differences could be observed in the expression pattern for both genes in comparison of atrium versus ventricle or left versus right heart (data not shown).

## Claims

1. A test system for the identification of an APJ receptor ligand comprising

(a) an APJ receptor or a functional variant thereof, and
(b) a G$\alpha$ protein which is selected from the following group: $G_{\alpha\ \Delta6qi4myr}$ protein, $G_{\alpha i4qi4}$ protein, $G_{\alpha16}$ protein

wherein the binding of said APJ receptor ligand to said APJ receptor or functional variant thereof effects a measurable or detectable signal.

**2.** The test system according to claim 1, wherein the ligand is an agonist.

**3.** The test system according to claim 2, wherein the agonist is a full, partial or inverse agonist.

**4.** The test system according to any of the claims 1, wherein the ligand is an antagonist.

**5.** The test system according to any of the claims 1 to 4, wherein the APJ receptor is a mammalian APJ receptor or a variant thereof.

**6.** The test system according to any of the claims 1 to 5, wherein the APJ receptor is the human, mouse or rat APJ receptor or a variant thereof.

**7.** The test system according to any of the claims 1 to 6, wherein the APJ receptor is the human APJ receptor or a variant thereof.

**8.** The test system according to any of the claims 1 to 7, wherein the signal is a change of $Ca^{2+}$ concentration.

**9.** The test system according to any of the claims 1 to 8, wherein the test system is located in a genetically engineered cell and wherein at least one of the components (a) or (b) is introduced into the cell.

**10.** The test system according to any of the claims 1 to 9, wherein the test system is located in a transient or stable transfected cell line or membrane thereof.

**11.** A method of screening an APJ receptor ligand, wherein the method comprises the steps of:

(a) providing the test system according to any of the proceeding claims,
(b) providing a test compound,
(c) optionally providing a known APJ receptor ligand, and
(d) measuring or detecting the influence of the test compound on the APJ receptor-mediated signal transduction pathway.

**12.** The method of screening according to claim 11, wherein the ligand is an agonist.

**13.** The method of screening according to claim 12, wherein the agonist is a full, partial or inverse agonist.

**14.** The method of screening according to claim 11, wherein the ligand is an antagonist.

**15.** The method according to any of the claims 11-14, wherein the test compound is provided in the form of a chemical compound library.

**16.** The method according to any of the claims 11 to 15, wherein the method is carried out using whole cells.

**17.** The method according to any of the claims 11 to 16, wherein the method is carried out in a robotics system.

**18.** The method according to any of the claims 11 to 17, wherein the method is a method of high-through put screening of an APJ receptor ligand.

**19.** A method for producing a medicament, wherein the method comprises the steps of:

(a) identifying a APJ receptor ligand carrying out the method according to any of the claims 11 to 18,
(b) providing adequate amounts of the ligand, and
(c) formulating the ligand with one or more pharmaceutically acceptable carriers or auxiliary substances.

**20.** A method for diagnosis or prognosis of a heart disease comprising the following steps:

(a) determining the expression level of APJ receptor gene and/or apelin gene in a sample to be tested,
(b) comparing the expression level of APJ receptor and/or apelin in the sample to be tested determined in step (a) with the expression level of APJ receptor and/or apelin in a control probe, and
(c) identifying a sample with increased APJ receptor and/or apelin gene expression.

**21.** The method according to claim 20, wherein the expression levels of the APJ receptor gene and/or apelin gene is/are determined on the basis of levels of mRNAs transcribed from the gene(s), or levels of polypeptides translated from the gene(s).

**22.** The method according to claim 20 or 21, wherein the levels of mRNAs are determined by a hybridization method or a nucleic acid amplification method.

**23.** The method according to claim 20 or 21, wherein the levels of polypeptides are determined by using binding proteins capable of binding specifically to the polypeptides or fragments thereof.

**24.** A kit usable for detection or prognosis of a heart disease, comprising primers and/or a probe which is usable for determining expression levels of the APJ receptor gene and/or apelin gene.

**25.** A kit usable for detection or prognosis of a heart disease, comprising antibodies capable of binding specifically to polypeptides encoded by the APJ receptor gene and/or apelin gene.

Fig.1

5′-BamHI GGATCC-ATGGGGTGCTGCCTGAGCGAGGAGGCCAAGGAAGCCCGGCGGATCAA
CGACGAG ATCGAGCGGCACGTCCGCAGGGACAAGCGGGACGCCCGCCGGGAGCTCAAGC
TGCTGCTGCTCGGGACAGGAGAGAGTGGCAAGAGTACGTTTATCAAGCAGATGAGAATCA
TCCATGGG TCAGGATACTCTGATGAAGATAAAAGGGGCTTCACCAAGCTGGTGTATCAGA
ACATCTTCACGGCCATGCAGGCCATGATCAGAGCCATGGACACACTCAAGATCCCATACA
AGTATGAG CACAATAAGGCTCATGCACAATTAGTTCGAGAAGTTGATGTGGAGAAGGTGT
CTGCTTTTGACGTCCCCGACTACGCGGCAATAAAGAGTTTATGGAATGATCCTGGAATCCA
GGAATGC TATGATAGACGACGAGAATATCAATTATCTGACTCTACCAAATACTATCTTAA
TGACTTG GACCGCGTAGCTGACCCTGCCTACCTGCCTACGCAACAAGATGTGCTTAGAGT
TCGAGTC CCCACCACAGGGATCATCGAATACCCCTTTGACTTACAAAGTGTCATTTTCAGA
ATGGTC GATGTAGGGGGCCAAAGGTCAGAGAGAAGAAAATGGATACACTGCTTTGAAAA
TGTCACC TCTATCATGTTTCTAGTAGCGCTTAGTGAATATGATCAAGTTCTCGTGGAGTCA
GACAAT GAGAACCGAATGGAGGAAAGCAAGGCTCTCTTTAGAACAATTATCACATACCCC
TGGTTC CAGAACTCCTCGGTTATTCTGTTCTTAAACAAGAAAGATCTTCTAGAGGAGAAAA
TCATG TATTCCCATCTAGTCGACTACTTCCCAGAATATGATGGACCCCAGAGAGATGCCCA
GGCA GCCCGAGAATTCATTCTGAAGATGTTCGTGGACCTGAACCCAGACAGTGACAAAA
TTATC TACTCCCACTTCACGTGCGCCACAGACACCGAGAATATCCGCTTTGTCTTTGCTGC
CGTC AAGGACACCATCCTCCAGTTGAACCTGAAGGAGtgtggcctcttcTAA-ATGCAT(NsiI)

Fig. 2

```
    CCACCGCTACCAGCGGTGGTTTGTTTGCCGGATCAAGAGCTACCAACTCTTTTTCCGAAG
  1 ---------+---------+---------+---------+---------+---------+ 60
    GGTGGCGATGGTCGCCACCAAACAAACGGCCTAGTTCTCGATGGTTGAGAAAAAGGCTTC

    GTAACTGGCTTCAGCAGAGCGCAGATACCAAATACTGTCCTTCTAGTGTAGCCGTAGTTA
 61 ---------+---------+---------+---------+---------+---------+ 120
    CATTGACCGAAGTCGTCTCGCGTCTATGGTTTATGACAGGAAGATCACATCGGCATCAAT

    GGCCACCACTTCAAGAACTCTGTAGCACCGCCTACATACCTCGCTCTGCTAATCCTGTTA
121 ---------+---------+---------+---------+---------+---------+ 180
    CCGGTGGTGAAGTTCTTGAGACATCGTGGCGGATGTATGGAGCGAGACGATTAGGACAAT

    CCAGTGGCTGCTGCCAGTGGCGATAAGTCGTGTCTTACCGGGTTGGACTCAAGACGATAG
181 ---------+---------+---------+---------+---------+---------+ 240
    GGTCACCGACGACGGTCACCGCTATTCAGCACAGAATGGCCCAACCTGAGTTCTGCTATC

    TTACCGGATAAGGCGCAGCGGTCGGGCTGAACGGGGGGTTCGTGCACACAGCCCAGCTTG
241 ---------+---------+---------+---------+---------+---------+ 300
    AATGGCCTATTCCGCGTCGCCAGCCCGACTTGCCCCCCAAGCACGTGTGTCGGGTCGAAC

    GAGCGAACGACCTACACCGAACTGAGATACCTACAGCGTGAGCATTGAGAAAGCGCCACG
301 ---------+---------+---------+---------+---------+---------+ 360
    CTCGCTTGCTGGATGTGGCTTGACTCTATGGATGTCGCACTCGTAACTCTTTCGCGGTGC

    CTTCCCGAAGGGAGAAAGGCGGACAGGTATCCGGTAAGCGGCAGGGTCGGAACAGGAGAG
361 ---------+---------+---------+---------+---------+---------+ 420
    GAAGGGCTTCCCTCTTTCCGCCTGTCCATAGGCCATTCGCCGTCCCAGCCTTGTCCTCTC

    CGCACGAGGGAGCTTCCAGGGGGAAACGCCTGGTATCTTTATAGTCCTGTCGGGTTTCGC
421 ---------+---------+---------+---------+---------+---------+ 480
    GCGTGCTCCCTCGAAGGTCCCCCTTTGCGGACCATAGAAATATCAGGACAGCCCAAAGCG

    CACCTCTGACTTGAGCGTCGATTTTTGTGATGCTCGTCAGGGGGGCGGAGCCTATGGAAA
481 ---------+---------+---------+---------+---------+---------+ 540
    GTGGAGACTGAACTCGCAGCTAAAAACACTACGAGCAGTCCCCCCGCCTCGGATACCTTT

    AACGCCAGCAACGCAAGCTAGAGTTTAAACTTGACAGATGAGACAATAACCCTGATAAAT
541 ---------+---------+---------+---------+---------+---------+ 600
    TTGCGGTCGTTGCGTTCGATCTCAAATTTGAACTGTCTACTCTGTTATTGGGACTATTTA

    GCTTCAATAATATTGAAAAAGGAAAAGTATGAGTATTCAACATTTCCGTGTCGCCCTTAT
601 ---------+---------+---------+---------+---------+---------+ 660
    CGAAGTTATTATAACTTTTTCCTTTTCATACTCATAAGTTGTAAAGGCACAGCGGGAATA

    TCCCTTTTTTGCGGCATTTTGCCTTCCTGTTTTTGCTCACCCAGAAACGCTGGTGAAAGT
661 ---------+---------+---------+---------+---------+---------+ 720
    AGGGAAAAAACGCCGTAAAACGGAAGGACAAAAACGAGTGGGTCTTTGCGACCACTTTCA

    AAAAGATGCAGAAGATCACTTGGGTGCGCGAGTGGGTTACATCGAACTGGATCTCAACAG
721 ---------+---------+---------+---------+---------+---------+ 780
    TTTTCTACGTCTTCTAGTGAACCCACGCGCTCACCCAATGTAGCTTGACCTAGAGTTGTC

    CGGTAAGATCCTTGAGAGTTTTCGCCCCGAAGAACGTTTCCCAATGATGAGCACTTTTAA
781 ---------+---------+---------+---------+---------+---------+ 840
    GCCATTCTAGGAACTCTCAAAAGCGGGGCTTCTTGCAAAGGGTTACTACTCGTGAAAATT

    AGTTCTGCTATGTGGCGCGGTATTATCCCGTATTCTTGGTTGAATACTCACCAGTCACAG
841 ---------+---------+---------+---------+---------+---------+ 900
    TCAAGACGATACACCGCGCCATAATAGGGCATAAGAACCAACTTATGAGTGGTCAGTGTC

    AAAAGCATCTTACGGATGGCATGACAGTAAGAGAATTATGCAGTGCTGCCATAACCATGA
901 ---------+---------+---------+---------+---------+---------+ 960
    TTTTCGTAGAATGCCTACCGTACTGTCATTCTCTTAATACGTCACGACGGTATTGGTACT

    GTGATAACACTGCGGCCAACTTACTTCTGACAACTATCGGAGGACCGAAGGAGCTAACCG
961 ---------+---------+---------+---------+---------+---------+ 1020
    CACTATTGTGACGCCGGTTGAATGAAGACTGTTGATAGCCTCCTGGCTTCCTCGATTGGC
```

```
     CTTTTTTGCACAACATGGGGGATCATGTAACTCGCCTTGATCGTTGGGAACCGGAGCTGA
1021 ---------+---------+---------+---------+---------+---------+ 1080
     GAAAAAACGTGTTGTACCCCCTAGTACATTGAGCGGAACTAGCAACCCTTGGCCTCGACT

     ATGAAGCCATACCAAACGACGAGCGTGACACCACGATGCCTGTAGCAATGGCAACAACGT
1081 ---------+---------+---------+---------+---------+---------+ 1140
     TACTTCGGTATGGTTTGCTGCTCGCACTGTGGTGCTACGGACATCGTTACCGTTGTTGCA

     TGCGAAAACTATTAACTGGCGAACTACTTACTCTAGCTTCCCGGCAACAACTAATAGACT
1141 ---------+---------+---------+---------+---------+---------+ 1200
     ACGCTTTTGATAATTGACCGCTTGATGAATGAGATCGAAGGGCCGTTGTTGATTATCTGA

     GGATGGAGGCGGATAAAGTTGCAGGACCACTTCTGCGCTCGGCACTTCCGGCTGGCTGGT
1201 ---------+---------+---------+---------+---------+---------+ 1260
     CCTACCTCCGCCTATTTCAACGTCCTGGTGAAGACGCGAGCCGTGAAGGCCGACCGACCA

     TTATTGCTGATAAATCAGGAGCCGGTGAGCGTGGGTCACGCGGTATCATTGCAGCACTGG
1261 ---------+---------+---------+---------+---------+---------+ 1320
     AATAACGACTATTTAGTCCTCGGCCACTCGCACCCAGTGCGCCATAGTAACGTCGTGACC

     GGCCGGATGGTAAGCCCTCCCGTATCGTAGTTATCTACACTACGGGGAGTCAGGCAACTA
1321 ---------+---------+---------+---------+---------+---------+ 1380
     CCGGCCTACCATTCGGGAGGGCATAGCATCAATAGATGTGATGCCCCTCAGTCCGTTGAT

     TGGATGAACGAAATAGACAGATCGCTGAGATAGGTGCCTCACTGATTAAGCATTGGTAAG
1381 ---------+---------+---------+---------+---------+---------+ 1440
     ACCTACTTGCTTTATCTGTCTAGCGACTCTATCCACGGAGTGACTAATTCGTAACCATTC

     GATAAATTTCTGGTAAGGAGGACACGTATGGAAGTGGGCAAGTTGGGGAAGCCGTATCCG
1441 ---------+---------+---------+---------+---------+---------+ 1500
     CTATTTAAAGACCATTCCTCCTGTGCATACCTTCACCCGTTCAACCCCTTCGGCATAGGC

     TTGCTGAATCTGGCATATGTGGGAGTATAAGACGCGCAGCGTCGCATCAGGCATTTTTTT
1501 ---------+---------+---------+---------+---------+---------+ 1560
     AACGACTTAGACCGTATACACCCTCATATTCTGCGCGTCGCAGCGTAGTCCGTAAAAAAA

     CTGCGCCAATGCAAAAAGGCCATCCGTCAGGATGGCCTTTCGCATAACTAGTGAGGCTCC
1561 ---------+---------+---------+---------+---------+---------+ 1620
     GACGCGGTTACGTTTTTCCGGTAGGCAGTCCTACCGGAAAGCGTATTGATCACTCCGAGG

     GGTGCCCGTCAGTGGGCAGAGCGCACATCGCCCACAGTCCCCGAGAAGTTGGGGGGAGGG
1621 ---------+---------+---------+---------+---------+---------+ 1680
     CCACGGGCAGTCACCCGTCTCGCGTGTAGCGGGTGTCAGGGGCTCTTCAACCCCCCTCCC

     GTCGGCAATTGAACCGGTGCCTAGAGAAGGTGGCGCGGGGTAAACTGGGAAAGTGATGTC
1681 ---------+---------+---------+---------+---------+---------+ 1740
     CAGCCGTTAACTTGGCCACGGATCTCTTCCACCGCGCCCCATTTGACCCTTTCACTACAG

     GTGTACTGGCTCCGCCTTTTTCCCGAGGGTGGGGGAGAACCGTATATAAGTGCAGTAGTC
1741 ---------+---------+---------+---------+---------+---------+ 1800
     CACATGACCGAGGCGGAAAAAGGGCTCCCACCCCCTCTTGGCATATATTCACGTCATCAG

     GCCGTGAACGTTCTTTTTCGCAACGGGTTTGCCGCCAGAACACAGGTAAGTGCCGTGTGT
1801 ---------+---------+---------+---------+---------+---------+ 1860
     CGGCACTTGCAAGAAAAAGCGTTGCCCAAACGGCGGTCTTGTGTCCATTCACGGCACACA

     GGTTCCCGCGGGCCTGGCCTCTTTACGGGTTATGGCCCTTGCGTGCCTTGAATTACTTCC
1861 ---------+---------+---------+---------+---------+---------+ 1920
     CCAAGGGCGCCCGGACCGGAGAAATGCCCAATACCGGGAACGCACGGAACTTAATGAAGG

     ACCTGGCTGCAGTACGTGATTCTTGATCCCGAGCTTCGGGTTGGAAGTGGGTGGGAGAGT
1921 ---------+---------+---------+---------+---------+---------+ 1980
     TGGACCGACGTCATGCACTAAGAACTAGGGCTCGAAGCCCAACCTTCACCCACCCTCTCA

     TCGAGGCCTTGCGCTTAAGGAGCCCCTTCGCCTCGTGCTTGAGTTGAGGCCTGGCCTGGG
1981 ---------+---------+---------+---------+---------+---------+ 2040
     AGCTCCGGAACGCGAATTCCTCGGGGAAGCGGAGCACGAACTCAACTCCGGACCGGACCC

     CGCTGGGGCCGCCGCGTGCGAATCTGGTGGCACCTTCGCGCCTGTCTCGCTGCTTTCGAT
2041 ---------+---------+---------+---------+---------+---------+ 2100
     GCGACCCCGGCGGCGCACGCTTAGACCACCGTGGAAGCGCGGACAGAGCGACGAAAGCTA
```

```
      AAGTCTCTAGCCATTTAAAATTTTTGATGACCTGCTGCGACGCTTTTTTTCTGGCAAGAT
2101  ---------+---------+---------+---------+---------+---------+ 2160
      TTCAGAGATCGGTAAATTTTAAAAACTACTGGACGACGCTGCGAAAAAAAGACCGTTCTA

      AGTCTTGTAAATGCGGGCCAAGATCGATCTGCACACTGGTATTTCGGTTTTTGGGGCCGC
2161  ---------+---------+---------+---------+---------+---------+ 2220
      TCAGAACATTTACGCCCGGTTCTAGCTAGACGTGTGACCATAAAGCCAAAAACCCCGGCG

      GGGCGGCGACGGGGCCCGTGCGTCCCAGCGCACATGTTCGGCGAGGCGGGGCCTGCGAGC
2221  ---------+---------+---------+---------+---------+---------+ 2280
      CCCGCCGCTGCCCCGGGCACGCAGGGTCGCGTGTACAAGCCGCTCCGCCCCGGACGCTCG

      GCGGCCACCGAGAATCGGACGGGGGTAGTCTCAAGCTGGCCGGCCTGCTCTGGTGCCTGG
2281  ---------+---------+---------+---------+---------+---------+ 2340
      CGCCGGTGGCTCTTAGCCTGCCCCCATCAGAGTTCGACCGGCCGGACGAGACCACGGACC

      CCTCGCGCCGCCGTGTATCGCCCCGCCCTGGGCGGCAAGGCTGGCCCGGTCGGCACCAGT
2341  ---------+---------+---------+---------+---------+---------+ 2400
      GGAGCGCGGCGGCACATAGCGGGGCGGGACCCGCCGTTCCGACCGGGCCAGCCGTGGTCA

      TGCGTGAGCGGAAAGATGGCCGCTTCCCGGCCCTGCTGCAGGGAGCTCAAAATGGAGGAC
2401  ---------+---------+---------+---------+---------+---------+ 2460
      ACGCACTCGCCTTTCTACCGGCGAAGGGCCGGGACGACGTCCCTCGAGTTTTACCTCCTG

      GCGGCGCTCGGGAGAGCGGGCGGGTGAGTCACCCACACAAAGGAAAAGGGCCTTTCCGTC
2461  ---------+---------+---------+---------+---------+---------+ 2520
      CGCCGCGAGCCCTCTCGCCCGCCCACTCAGTGGGTGTGTTTCCTTTTCCCGGAAAGGCAG

      CTCAGCCGTCGCTTCATGTGACTCCACGGAGTACCGGGCGCCGTCCAGGCACCTCGATTA
2521  ---------+---------+---------+---------+---------+---------+ 2580
      GAGTCGGCAGCGAAGTACACTGAGGTGCCTCATGGCCCGCGGCAGGTCCGTGGAGCTAAT

     XhoI
       |
      GTTCTCGAGCTTTTGGAGTACGTCGTCTTTAGGTTGGGGGGAGGGGTTTTATGCGATGGA
2581  ---------+---------+---------+---------+---------+---------+ 2640
      CAAGAGCTCGAAAACCTCATGCAGCAGAAATCCAACCCCCCTCCCCAAAATACGCTACCT

              DraIII
      GTTTCCCCACACTGAGTGGGTGGAGACTGAAGTTAGGCCAGCTTGGCACTTGATGTAATT
2641  ---------+---------+---------+---------+---------+---------+ 2700
      CAAAGGGGTGTGACTCACCCACCTCTGACTTCAATCCGGTCGAACCGTGAACTACATTAA

      CTCCTTGGAATTTGCCCTTTTTGAGTTTGGATCTTGGTTCATTCTCAAGCCTCAGACAGT
2701  ---------+---------+---------+---------+---------+---------+ 2760
      GAGGAACCTTAAACGGGAAAAACTCAAACCTAGAACCAAGTAAGAGTTCGGAGTCTGTCA

                                             HindIII
      GGTTCAAAGTTTTTTTCTTCCATTTCAGGTGTCGTGAAAAGCTTGCCACCATGGACTACA
2761  ---------+---------+---------+---------+---------+---------+ 2820
      CCAAGTTTCAAAAAAAGAAGGTAAAGTCCACAGCACTTTTCGAACGGTGGTACCTGATGT

                                    recently EcoRI
      AGGACGACGACGACAAGAACATGTGGGGCGAATTAATTCCTTAAGCTATCAACAAGTTTG
2821  ---------+---------+---------+---------+---------+---------+ 2880
      TCCTGCTGCTGCTGTTCTTGTACACCCCGCTTAATTAAgGAATTCGATAGTTGTTCAAAC

      TACAAAAAAGCAGGCTTAGGAATGGAGGAAGGTGGTGATTTTGACAACTACTATGGGGCA
2881  ---------+---------+---------+---------+---------+---------+ 2940
      ATGTTTTTTCGTCCGAATCCTTACCTCCTTCCACCACTAAAACTGTTGATGATACCCCGT

                           M  E  E  G  G  D  F  D  N  Y  Y  G  A  -

      GACAACCAGTCTGAGTGTGAGTACACAGACTGGAAATCCTCGGGGGCCCTCATCCCTGCC
2941  ---------+---------+---------+---------+---------+---------+ 3000
      CTGTTGGTCAGACTCACACTCATGTGTCTGACCTTTAGGAGCCCCCGGGAGTAGGGACGG
```

```
      D  N  Q  S  E  C  E  Y  T  D  W  K  S  S  G  A  L  I  P  A   -

     ATCTACATGTTGGTCTTCCTCCTGGGCACCACGGGAAACGGTCTGGTGCTCTGGACCGTG
3001 ---------+---------+---------+---------+---------+---------+ 3060
     TAGATGTACAACCAGAAGGAGGACCCGTGGTGCCCTTTGCCAGACCACGAGACCTGGCAC

      I  Y  M  L  V  F  L  L  G  T  T  G  N  G  L  V  L  W  T  V   -

                                         EcoRV
     TTTCGGAGCAGCCGGGAGAAGAGGCGCTCAGCTGATATCTTCATTGCTAGCCTGGCGGTG
3061 ---------+---------+---------+---------+---------+---------+ 3120
     AAAGCCTCGTCGGCCCTCTTCTCCGCGAGTCGACTATAGAAGTAACGATCGGACCGCCAC

     F  R  S  S  R  E  K  R  R  S  A  D  I  F  I  A  S  L  A  V   -

     GCTGACCTGACCTTCGTGGTGACGCTGCCCCTGTGGGCTACCTACACGTACCGGGACTAT
3121 ---------+---------+---------+---------+---------+---------+ 3180
     CGACTGGACTGGAAGCACCACTGCGACGGGGACACCCGATGGATGTGCATGGCCCTGATA

      A  D  L  T  F  V  V  T  L  P  L  W  A  T  Y  T  Y  R  D  Y   -

     GACTGGCCCTTTGGGACCTTCTTCTGCAAGCTCAGCAGCTACCTCATCTTCGTCAACATG
3181 ---------+---------+---------+---------+---------+---------+ 3240
     CTGACCGGGAAACCCTGGAAGAAGACGTTCGAGTCGTCGATGGAGTAGAAGCAGTTGTAC

      D  W  P  F  G  T  F  F  C  K  L  S  S  Y  L  I  F  V  N  M   -

     TACGCCAGCGTCTTCTGCCTCACCGGCCTCAGCTTCGACCGCTACCTGGCCATCGTGAGG
3241 ---------+---------+---------+---------+---------+---------+ 3300
     ATGCGGTCGCAGAAGACGGAGTGGCCGGAGTCGAAGCTGGCGATGGACCGGTAGCACTCC

      Y  A  S  V  F  C  L  T  G  L  S  F  D  R  Y  L  A  I  V  R   -

     CCAGTGGCCAATGCTCGGCTGAGGCTGCGGGTCAGCGGGGCCGTGGCCACGGCAGTTCTT
3301 ---------+---------+---------+---------+---------+---------+ 3360
     GGTCACCGGTTACGAGCCGACTCCGACGCCCAGTCGCCCCGGCACCGGTGCCGTCAAGAA

      P  V  A  N  A  R  L  R  L  R  V  S  G  A  V  A  T  A  V  L   -

     TGGGTGCTGGCCGCCCTCCTGGCCATGCCTGTCATGGTGTTACGCACCACCGGGGACTTG
3361 ---------+---------+---------+---------+---------+---------+ 3420
     ACCCACGACCGGCGGGAGGACCGGTACGGACAGTACCACAATGCGTGGTGGCCCCTGAAC

      W  V  L  A  A  L  L  A  M  P  V  M  V  L  R  T  T  G  D  L   -

     GAGAACACCACTAAGGTGCAGTGCTACATGGACTACTCCATGGTGGCCACTGTGAGCTCA
3421 ---------+---------+---------+---------+---------+---------+ 3480
     CTCTTGTGGTGATTCCACGTCACGATGTACCTGATGAGGTACCACCGGTGACACTCGAGT

      E  N  T  T  K  V  Q  C  Y  M  D  Y  S  M  V  A  T  V  S  S   -

     GAGTGGGCCTGGGAGGTGGGCCTTGGGGTCTCGTCCACCACCGTGGGCTTTGTGGTGCCC
3481 ---------+---------+---------+---------+---------+---------+ 3540
     CTCACCCGGACCCTCCACCCGGAACCCCAGAGCAGGTGGTGGCACCCGAAACACCACGGG

      E  W  A  W  E  V  G  L  G  V  S  S  T  T  V  G  F  V  V  P   -

     TTCACCATCATGCTGACCTGTTACTTCTTCATCGCCCAAACCATCGCTGGCCACTTCCGC
3541 ---------+---------+---------+---------+---------+---------+ 3600
     AAGTGGTAGTACGACTGGACAATGAAGAAGTAGCGGGTTTGGTAGCGACCGGTGAAGGCG
```

```
F   T   I   M   L   T   C   Y   F   F   I   A   Q   T   I   A   G   H   F   R    -

     AAGGAACGCATCGAGGGCCTGCGGAAGCGGCGCCGGCTGCTCAGCATCATCGTGGTGCTG
3601 ---------+---------+---------+---------+---------+---------+ 3660
     TTCCTTGCGTAGCTCCCGGACGCCTTCGCCGCGGCCGACGAGTCGTAGTAGCACCACGAC

K   E   R   I   E   G   L   R   K   R   R   R   L   L   S   I   I   V   V   L    -

                                              DraIII
     GTGGTGACCTTTGCCCTGTGCTGGATGCCCTACCACCTGGTGAAGACGCTGTACATGCTG
3661 ---------+---------+---------+---------+---------+---------+ 3720
     CACCACTGGAAACGGGACACGACCTACGGGATGGTGGACCACTTCTGCGACATGTACGAC

V   V   T   F   A   L   C   W   M   P   Y   H   L   V   K   T   L   Y   M   L    -

     GGCAGCCTGCTGCACTGGCCCTGTGACTTTGACCTCTTCCTCATGAACATCTTCCCCTAC
3721 ---------+---------+---------+---------+---------+---------+ 3780
     CCGTCGGACGACGTGACCGGGACACTGAAACTGGAGAAGGAGTACTTGTAGAAGGGGATG

G   S   L   L   H   W   P   C   D   F   D   L   F   L   M   N   I   F   P   Y    -

     TGCACCTGCATCAGCTACGTCAACAGCTGCCTCAACCCCTTCCTCTATGCCTTTTTCGAC
3781 ---------+---------+---------+---------+---------+---------+ 3840
     ACGTGGACGTAGTCGATGCAGTTGTCGACGGAGTTGGGGAAGGAGATACGGAAAAAGCTG

C   T   C   I   S   Y   V   N   S   C   L   N   P   F   L   Y   A   F   F   D    -

     CCCCGCTTCCGCCAGGCCTGCACCTCCATGCTCTGCTGTGGCCAGAGCAGGTGCGCAGGC
3841 ---------+---------+---------+---------+---------+---------+ 3900
     GGGGCGAAGGCGGTCCGGACGTGGAGGTACGAGACGACACCGGTCTCGTCCACGCGTCCG

P   R   F   R   Q   A   C   T   S   M   L   C   C   G   Q   S   R   C   A   G    -

     ACCTCCCACAGCAGCAGTGGGGAGAAGTCAGCCAGCTACTCTTCGGGGCACAGCCAGGGG
3901 ---------+---------+---------+---------+---------+---------+ 3960
     TGGAGGGTGTCGTCGTCACCCCTCTTCAGTCGGTCGATGAGAAGCCCCGTGTCGGTCCCC

T   S   H   S   S   S   G   E   K   S   A   S   Y   S   S   G   H   S   Q   G    -

     CCCGGCCCCAACATGGGCAAGGGTGGAGAACAGATGCACGAGAAATCCATCCCCTACAGC
3961 ---------+---------+---------+---------+---------+---------+ 4020
     GGGCCGGGGTTGTACCCGTTCCCACCTCTTGTCTACGTGCTCTTTAGGTAGGGGATGTCG

P   G   P   N   M   G   K   G   G   E   Q   M   H   E   K   S   I   P   Y   S    -

     CAGGAGACCCTTGTGGTTGACTAGGACCCAGCTTTCTTGTACAAAGTGGTTGATAGCTTG
4021 ---------+---------+---------+---------+---------+---------+ 4080
     GTCCTCTGGGAACACCAACTGATCCTGGGTCGAAAGAACATGTTTCACCAACTATCGAAC

Q   E   T   L   V   V   D   *

          BamHI                          recently EcoRI
     GTACCGAGCTCGGATCCACTAGTCCAGTGTGGTGGAATTGGCCGCAGGTAAGCCAGCCCA
4081 ---------+---------+---------+---------+---------+---------+ 4140
     CATGGCTCGAGCCTAGGTGATCAGGTCACACCACCTTAACCGGCGTCCATTCGGTCGGGT

     GGCCTCGCCCTCCAGCTCAAGGCGGGACAGGTGCCCTAGAGTAGCCTGCATCCAGGGACA
4141 ---------+---------+---------+---------+---------+---------+ 4200
     CCGGAGCGGGAGGTCGAGTTCCGCCCTGTCCACGGGATCTCATCGGACGTAGGTCCCTGT

     GGCCCCAGCCGGGTGCTGACACGTCCACCTCCATCTCTTCCTCAGGTCTGCCCGGGTGGC
4201 ---------+---------+---------+---------+---------+---------+ 4260
     CCGGGGTCGGCCCACGACTGTGCAGGTGGAGGTAGAGAAGGAGTCCAGACGGGCCCACCG
```

```
     ATCCCTGTGACCCCTCCCCAGTGCCTCTCCTGGCCCTGGAAGTTGCCACTCCAGTGCCCA
4261 ---------+---------+---------+---------+---------+---------+ 4320
     TAGGGACACTGGGGAGGGGTCACGGAGAGGACCGGGACCTTCAACGGTGAGGTCACGGGT

     CCAGCCTTGTCCTAATAAAATTAAGTTGCATCATTTTGTCTGACTAGGTGTCCTTCTATA
4321 ---------+---------+---------+---------+---------+---------+ 4380
     GGTCGGAACAGGATTATTTTAATTCAACGTAGTAAAACAGACTGATCCACAGGAAGATAT

     ATATTATGGGGTGGAGGGGGGTGGTATGGAGCAAGGGGCCCAAGTTAACTTGTTTATTGC
4381 ---------+---------+---------+---------+---------+---------+ 4440
     TATAATACCCCACCTCCCCCCACCATACCTCGTTCCCCGGGTTCAATTGAACAAATAACG

     AGCTTATAATGGTTACAAATAAAGCAATAGCATCACAAATTTCACAAATAAAGCATTTTT
4441 ---------+---------+---------+---------+---------+---------+ 4500
     TCGAATATTACCAATGTTTATTTCGTTATCGTAGTGTTTAAAGTGTTTATTTCGTAAAAA

                                                          BamHI
     TTCACTGCATTCTAGTTGTGGTTTGTCCAAACTCATCAATGTATCTTATCATGTCTGGAT
4501 ---------+---------+---------+---------+---------+---------+ 4560
     AAGTGACGTAAGATCAACACCAAACAGGTTTGAGTAGTTACATAGAATAGTACAGACCTA

     CCGCTTCAGGCACCGGGCTTGCGGGTCATGCACCAGGTCGCGCGGTCCTTCGGGCACTCG
4561 ---------+---------+---------+---------+---------+---------+ 4620
     GGCGAAGTCCGTGGCCCGAACGCCCAGTACGTGGTCCAGCGCGCCAGGAAGCCCGTGAGC

     ACGTCGGCGGTGACGGTGAAGCCGAGCCGCTCGTAGAAGGGGAGGTTGCGGGGCGCGGAG
4621 ---------+---------+---------+---------+---------+---------+ 4680
     TGCAGCCGCCACTGCCACTTCGGCTCGGCGAGCATCTTCCCCTCCAACGCCCCGCGCCTC

     GTCTCCAGGAAGGCGGGCACCCCGGCGCGCTCGGCCGCCTCCACTCCGGGGAGCACGACG
4681 ---------+---------+---------+---------+---------+---------+ 4740
     CAGAGGTCCTTCCGCCCGTGGGGCCGCGCGAGCCGGCGGAGGTGAGGCCCCTCGTGCTGC

     GCGCTGCCCAGACCCTTGCCCTGGTGGTCGGGCGAGACGCCGACGGTGGCCAGGAACCAC
4741 ---------+---------+---------+---------+---------+---------+ 4800
     CGCGACGGGTCTGGGAACGGGACCACCAGCCCGCTCTGCGGCTGCCACCGGTCCTTGGTG

     GCGGGCTCCTTGGGCCGGTGCGGCGCCAGGAGGCCTTCCATCTGTTGCTGCGCGGCCAGC
4801 ---------+---------+---------+---------+---------+---------+ 4860
     CGCCCGAGGAACCCGGCCACGCCGCGGTCCTCCGGAAGGTAGACAACGACGCGCCGGTCG

     CGGGAACCGCTCAACTCGGCCATGCGCGGGCCGATCTCGGCGAACACCGCCCCCGCTTCG
4861 ---------+---------+---------+---------+---------+---------+ 4920
     GCCCTTGGCGAGTTGAGCCGGTACGCGCCCGGCTAGAGCCGCTTGTGGCGGGGGCGAAGC

     ACGCTCTCCGGCGTGGTCCAGACCGCCACCGCGGCGCCGTCGTCCGCGACCCACACCTTG
4921 ---------+---------+---------+---------+---------+---------+ 4980
     TGCGAGAGGCCGCACCAGGTCTGGCGGTGGCGCCGCGGCAGCAGGCGCTGGGTGTGGAAC

   CCGATGTCGAGCCCGACGCGCGTGAGGAAGAGTTCTTGCAGCTCGGTGACCCGCTCGATG
4981 ---------+---------+---------+---------+---------+---------+ 5040
     GGCTACAGCTCGGGCTGCGCGCACTCCTTCTCAAGAACGTCGAGCCACTGGGCGAGCTAC

     TGGCGGTCCGGGTCGACGGTGTGGCGCGTGGCGGGGTAGTCGGCGAACGCGGCGGCGAGG
5041 ---------+---------+---------+---------+---------+---------+ 5100
     ACCGCCAGGCCCAGCTGCCACACCGCGCACCGCCCCATCAGCCGCTTGCGCCGCCGCTCC

     GTGCGTACGGCCCGGGGGACGTCGTCGCGGGTGGCGAGGCGCACCGTGGGCTTGTACTCG
5101 ---------+---------+---------+---------+---------+---------+ 5160
     CACGCATGCCGGGCCCCCTGCAGCAGCGCCCACCGCTCCGCGTGGCACCCGAACATGAGC

     GTCATGGTGGCCTGCAGAGTCGCTCGGTGTTCGAGGCCACACGCGTCACCTTAATATGCG
5161 ---------+---------+---------+---------+---------+---------+ 5220
     CAGTACCACCGGACGTCTCAGCGAGCCACAAGCTCCGGTGTGCGCAGTGGAATTATACGC

     AAGTGGACCTGGGACCGCGCCGCCCCGACTGCATCTGCGTGTTAATTCGCCAATGACAAG
5221 ---------+---------+---------+---------+---------+---------+ 5280
     TTCACCTGGACCCTGGCGCGGCGGGGCTGACGTAGACGCACAATTAAGCGGTTACTGTTC

     ACGCTGGGCGGGGTTTGTGTCATCATAGAACTAAAGACATGCAAATATATTTCTTCCGGG
```

```
5281 ---------+---------+---------+---------+---------+---------+ 5340
     TGCGACCCGCCCCAAACACAGTAGTATCTTGATTTCTGTACGTTTATATAAAGAAGGCCC

     GACACCGCCAGCAAACGCGAGCAACGGGCCACGGGGATGAAGCAGCTGCGCCACTCCCTG
5341 ---------+---------+---------+---------+---------+---------+ 5400
     CTGTGGCGGTCGTTTGCGCTCGTTGCCCGGTGCCCCTACTTCGTCGACGCGGTGAGGGAC

     AAGATCCATCGTCTCCTAACAAGTTACATCACTCCTGCCCTTCCTCACCCTCATCTCCAT
5401 ---------+---------+---------+---------+---------+---------+ 5460
     TTCTAGGTAGCAGAGGATTGTTCAATGTAGTGAGGACGGGAAGGAGTGGGAGTAGAGGTA

     CACCTCCTTCATCTCCGTCATCTCCGTCATCACCCTCCGCGGCAGCCCCTTCCACCATAG
5461 ---------+---------+---------+---------+---------+---------+ 5520
     GTGGAGGAAGTAGAGGCAGTAGAGGCAGTAGTGGGAGGCGCCGTCGGGGAAGGTGGTATC

     GTGGAAACCAGGGAGGCAAATCTACTCCATCGTCAAAGCTGCACACAGTCACCCTGATAT
5521 ---------+---------+---------+---------+---------+---------+ 5580
     CACCTTTGGTCCCTCCGTTTAGATGAGGTAGCAGTTTCGACGTGTGTCAGTGGGACTATA

     TGCAGGTAGGAGCGGGCTTTGTCATAACAAGGTCCTTAATCGCATCCTTCAAAACCTCAG
5581 ---------+---------+---------+---------+---------+---------+ 5640
     ACGTCCATCCTCGCCCGAAACAGTATTGTTCCAGGAATTAGCGTAGGAAGTTTTGGAGTC

     CAAATATATGAGTTTGTAAAAAGACCATGAAATAACAGACAATGGACTCCCTTAGCGGGC
5641 ---------+---------+---------+---------+---------+---------+ 5700
     GTTTATATACTCAAACATTTTTCTGGTACTTTATTGTCTGTTACCTGAGGGAATCGCCCG

     CAGGTTGTGGGCCGGGTCCAGGGGCCATTCCAAAGGGGAGACGACTCAATGGTGTAAGAC
5701 ---------+---------+---------+---------+---------+---------+ 5760
     GTCCAACACCCGGCCCAGGTCCCCGGTAAGGTTTCCCCTCTGCTGAGTTACCACATTCTG

     GACATTGTGGAATAGCAAGGGCAGTTCCTCGCCTTAGGTTGTAAAGGGAGGTCTTACTAC
5761 ---------+---------+---------+---------+---------+---------+ 5820
     CTGTAACACCTTATCGTTCCCGTCAAGGAGCGGAATCCAACATTTCCCTCCAGAATGATG

     CTCCATATACGAACACACCGGCGACCCAAGTTCCTTCGTCGGTAGTCCTTTCTACGTGAC
5821 ---------+---------+---------+---------+---------+---------+ 5880
     GAGGTATATGCTTGTGTGGCCGCTGGGTTCAAGGAAGCAGCCATCAGGAAAGATGCACTG

     TCCTAGCCAGGAGAGCTCTTAAACCTTCTGCAATGTTCTCAAATTTCGGGTTGGAACCTC
5881 ---------+---------+---------+---------+---------+---------+ 5940
     AGGATCGGTCCTCTCGAGAATTTGGAAGACGTTACAAGAGTTTAAAGCCCAACCTTGGAG

     CTTGACCACGATGCTTTCCAAACCACCCTCCTTTTTTGCGCCTGCCTCCATCACCCTGAC
5941 ---------+---------+---------+---------+---------+---------+ 6000
     GAACTGGTGCTACGAAAGGTTTGGTGGGAGGAAAAAACGCGGACGGAGGTAGTGGGACTG

     CCCCGCTGCGCGGGGGCACGTCAGGCTCACCATCTGGGCCGCCTTCTTGGTGGTATTCAA
6001 ---------+---------+---------+---------+---------+---------+ 6060
     GGGGCGACGCGCCCCCGTGCAGTCCGAGTGGTAGACCCGGCGGAAGAACCACCATAAGTT

     AATAATCGGCTTCCCCTACAGGGTGGAAAAATGGCCTTCTACCTGGAGGGGGCCTGCGCG
6061 ---------+---------+---------+---------+---------+---------+ 6120
     TTATTAGCCGAAGGGGATGTCCCACCTTTTTACCGGAAGATGGACCTCCCCCGGACGCGC

     GTGGAGACCCGGATGATGATGACTGACTACTGGGACTCCTGGGCCTCTTTTCTCCACGTC
6121 ---------+---------+---------+---------+---------+---------+ 6180
     CACCTCTGGGCCTACTACTACTGACTGATGACCCTGAGGACCCGGAGAAAAGAGGTGCAG

     CACGACCTCTCCCCCTGGCTCTTTCACGACTTCCCCCCCTGGCTCTTTCACGTCCTCTAC
6181 ---------+---------+---------+---------+---------+---------+ 6240
     GTGCTGGAGAGGGGGACCGAGAAAGTGCTGAAGGGGGGGACCGAGAAAGTGCAGGAGATG

     CCCGGCGGCCTCCACTACCTCCTCGACCCCGGCCTCCACTACCTCCTCGACCCCGGCCTC
6241 ---------+---------+---------+---------+---------+---------+ 6300
     GGGCCGCCGGAGGTGATGGAGGAGCTGGGGCCGGAGGTGATGGAGGAGCTGGGGCCGGAG

     CACTGCCTCCTCGACCCCGGCCTCCACCTCCTGCTCCTGCCCCTCCCGCTCCTGCTCCTG
6301 ---------+---------+---------+---------+---------+---------+ 6360
     GTGACGGAGGAGCTGGGGCCGGAGGTGGAGGACGAGGACGGGGAGGGCGAGGACGAGGAC
```

```
     CTCCTGTTCCACCGTGGGTCCCTTTGCAGCCAATGCAACTTGGACGTTTTTGGGGTCTCC
6361 ---------+---------+---------+---------+---------+---------+ 6420
     GAGGACAAGGTGGCACCCAGGGAAACGTCGGTTACGTTGAACCTGCAAAAACCCCAGAGG

     GGACACCATCTCTATGTCTTGGCCCTGATCCTGAGCCGCCCGGGGCTCCTGGTCTTCCGC
6421 ---------+---------+---------+---------+---------+---------+ 6480
     CCTGTGGTAGAGATACAGAACCGGGACTAGGACTCGGCGGGCCCCGAGGACCAGAAGGCG

     CTCCTCGTCCTCGTCCTCTTCCCCGTCCTCGTCCATGTGCCATGATGGCGGCCTGCAGCT
6481 ---------+---------+---------+---------+---------+---------+ 6540
     GAGGAGCAGGAGCAGGAGAAGGGGCAGGAGCAGGTACACGGTACTACCGCCGGACGTCGA

     GTGTTCGAGGCCGCGCGTGTCACCTTAATATGCGAAGTGGACCTGGGACCGCGCCGCCCC
6541 ---------+---------+---------+---------+---------+---------+ 6600
     CACAAGCTCCGGCGCGCACAGTGGAATTATACGCTTCACCTGGACCCTGGCGCGGCGGGG

     GACTGCATCTGCGTGTTCGAGTTCGCCAATGACAAGACGCTGGGCGGGGAGATCCCCCTT
6601 ---------+---------+---------+---------+---------+---------+ 6660
     CTGACGTAGACGCACAAGCTCAAGCGGTTACTGTTCTGCGACCCGCCCCTCTAGGGGGAA

     ATTAACCCTAAACGGGTAGCATATGCTTCCCGGGTAGTAGTATATACTATCCAGACTAAC
6661 ---------+---------+---------+---------+---------+---------+ 6720
     TAATTGGGATTTGCCCATCGTATACGAAGGGCCCATCATCATATATGATAGGTCTGATTG

     CCTAATTCAATAGCATATGTTACCCAACGGGAAGCATATGCTATCGAATTAGGGTTAGTA
6721 ---------+---------+---------+---------+---------+---------+ 6780
     GGATTAAGTTATCGTATACAATGGGTTGCCCTTCGTATACGATAGCTTAATCCCAATCAT

     AAAGGGTCCTAAGGAACAGCGATCTGGATAGCATATGCTATCCTAATCTATATCTGGGTA
6781 ---------+---------+---------+---------+---------+---------+ 6840
     TTTCCCAGGATTCCTTGTCGCTAGACCTATCGTATACGATAGGATTAGATATAGACCCAT

     GCATATGCTATCCTAATCTATATCTGGGTAGCATAGGCTATCCTAATCTATATCTGGGTA
6841 ---------+---------+---------+---------+---------+---------+ 6900
     CGTATACGATAGGATTAGATATAGACCCATCGTATCCGATAGGATTAGATATAGACCCAT

     GCATATGCTATCCTAATCTATATCTGGGTAGTATATGCTATCCTAATTTATATCTGGGTA
6901 ---------+---------+---------+---------+---------+---------+ 6960
     CGTATACGATAGGATTAGATATAGACCCATCATATACGATAGGATTAAATATAGACCCAT

     GCATAGGCTATCCTAATCTATATCTGGGTAGCATATGCTATCCTAATCTATATCTGGGTA
6961 ---------+---------+---------+---------+---------+---------+ 7020
     CGTATCCGATAGGATTAGATATAGACCCATCGTATACGATAGGATTAGATATAGACCCAT

     GTATATGCTATCCTAATCTGTATCCGGGTAGCATATGCTATCCTCATGCATATACAGTCA
7021 ---------+---------+---------+---------+---------+---------+ 7080
     CATATACGATAGGATTAGACATAGGCCCATCGTATACGATAGGAGTACGTATATGTCAGT

     GCATATGATACCCAGTAGTAGAGTGGGAGTGCTATCCTTTGCATATGCCGCCACCTCCCA
7081 ---------+---------+---------+---------+---------+---------+ 7140
     CGTATACTATGGGTCATCATCTCACCCTCACGATAGGAAACGTATACGGCGGTGGAGGGT

     AGGAGATCTGTCGACATCGATGGGCGCGGGTGTACACTCCGCCCATCCCGCCCCTAACTC
7141 ---------+---------+---------+---------+---------+---------+ 7200
     TCCTCTAGACAGCTGTAGCTACCCGCGCCCACATGTGAGGCGGGTAGGGCGGGGATTGAG

     CGCCCAGTTCCGCCCATTCTCCGCCTCATGGCTGACTAATTTTTTTTATTTATGCAGAGG
7201 ---------+---------+---------+---------+---------+---------+ 7260
     GCGGGTCAAGGCGGGTAAGAGGCGGAGTACCGACTGATTAAAAAAAATAAATACGTCTCC

     CCGAGGCCGCCTCGGCCTCTGAGCTATTCCAGAAGTAGTGAGGAGGCTTTTTTGGAGGCC
7261 ---------+---------+---------+---------+---------+---------+ 7320
     GGCTCCGGCGGAGCCGGAGACTCGATAAGGTCTTCATCACTCCTCCGAAAAAACCTCCGG

     TAGGCTTTTGCAAAAAGCTAATTC
7321 ---------+---------+---- 7344
     ATCCGAAAACGTTTTTCGATTAAG
```

Fig. 3

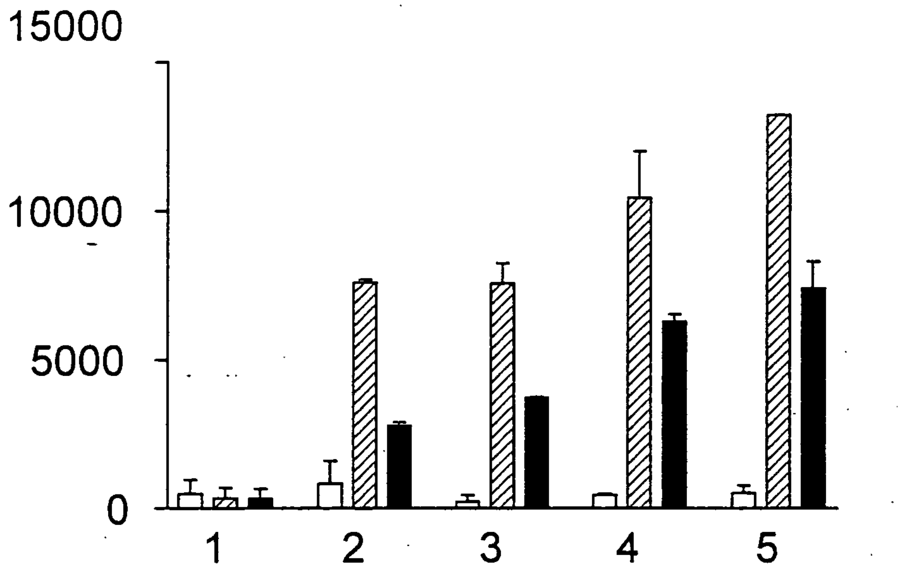

Fig. 4

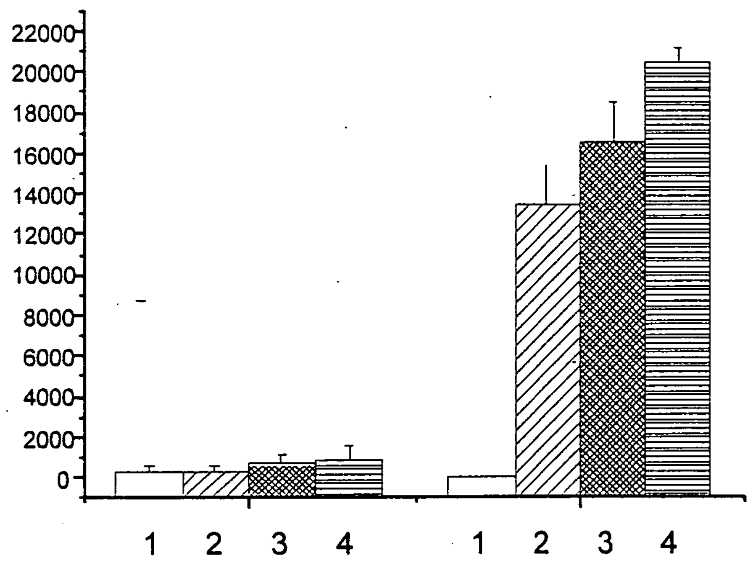
22000
20000
18000
16000
14000
12000
10000
8000
6000
4000
2000
0
1
2
3
4
1
2
3
4

A B

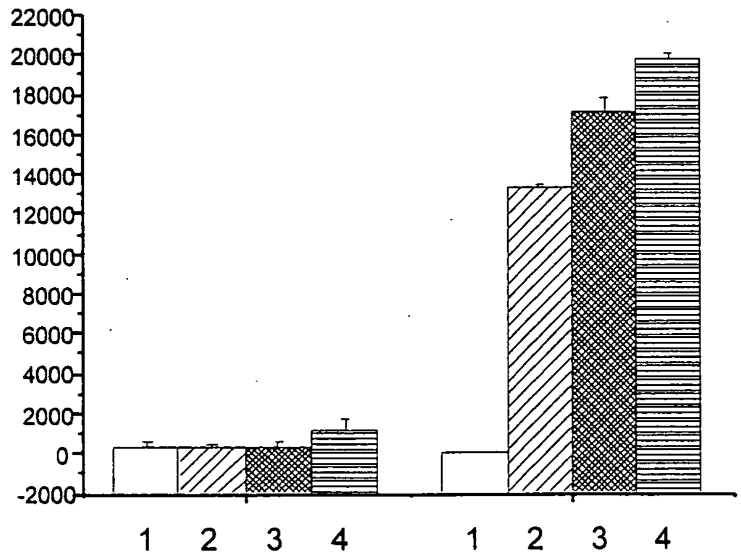
22000
20000
18000
16000
14000
12000
10000
8000
6000
4000
2000
0
-2000
1
2
3
4
1
2
3
4

A B

Fig. 5

```
  1 MGCCLSEEAK EARRINDEIE RHVRRDKRDA RRELKLLLLG TGESGKSTFI
 51 KQMRIIHGSG YSDEDKRGFT KLVYQNIFTA MQAMIRAMDT LKIPYKYEHN
101 KAHAQLVREV DVEKVSAFDV PDYAAIKSLW NDPGIQECYD RRREYQLSDS
151 TKYYLNDLDR VADPAYLPTQ QDVLRVRVPT TGIIEYPFDL QSVIFRMVDV
201 GGQRSERRKW IHCFENVTSI MFLVALSEYD QVLVESDNEN RMEESKALFR
251 TIITYPWFQN SSVILFLNKK DLLEEKIMYS HLVDYFPEYD GPQRDAQAAR
301 EFILKMFVDL NPDSDKIIYS HFTCATDTEN IRFVFAAVKD TILQLNLKEC
    GLF*
```

Fig. 6

```
AgatccATGGGGTGCACCCTGAGCGAGGAGGCCAAGGAAGCCCGGAGGATCAACG
ACGAGATCGAGCGGCAGCTGCGCAGGGACAAGCGCGACGCCCGCCGGGAGCTCAA
GCTGCTGCTGCTGGGGACAGGGGAGAGTGGCAAGTCGACCTTCATCAAGCAGATG
AGGATCATCCACGGGTCGGACTACTCTGACGAAGACAAGCGCGGCTTCACCAAGC
TGGTGTATCAGAACATCTTCACGGCCATGCAGGCCATGATCAGAGCGATGGACAC
ACTCAAGATCCCATACAAGTATGAACACAATAAGGCTCATGCACAATTGGTTCGA
GAGGTTGATGTGGAGAAGGTGTCTGCTTTTGACGTCCCCGACTACGCGGCAATAA
AGAGCTTGTGGAATGATCCTGGAATCCAGGAGTGCTACGACAGACGACGGAATA
TCAGTTATCTGACTCTACCAAATACTATCTGAATGACTTGGACCGTGTAGCCGAC
CCTTCCTATCTGCCTACACAACAAGACGTGCTTAGAGTTCGAGTCCCCACTACAG
GGATCATCGAATACCCCTTTGACTTACAAAGTGTCATTTTCAGAATGGTCGATGT
AGGGGGCCAAAGGTCAGAGAGAAGAAAATGGATACACTGCTTTGAAAATGTCACC
TCCATCATGTTTCTAGTAGCGCTTAGCGAATATGATCAAGTTCTTGTGGAGTCAG
ACAATGAGAACCGCATGGAGGAGAGCAAAGCACTCTTTAGAACAATTATCACCTA
CCCCTGGTTCCAGAACTCCTCTGTGATTCTGTTCTTAAACAAGAAAGATCTTCTA
GAGGAGAAAATCATGTATTCCCACCTAGTCGACTACTTCCCAGAATATGATGGAC
CCCAGAGAGATGCCCAGGCAGCTCGAGAATTCATCCTGAAAATGTTCGTGGACCT
GAACCCCGACAGTGACAAAATCATCTACTCCCACTTCACGTGCGCCACAGATACC
GAGAACATCCGCTTCGTCTTTGCAGCCGTCAAGGACACCATCCTGCAGCTGAACC
TGAAGGAGTGTGGCCTCTTCTGAgataaa
```

Fig. 7

```
  1 MGCTLSEEAK EARRINDEIE RQLRRDKRDA RRELKLLLLG TGESGKSTFI
 51 KQMRIIHGSD YSDEDKRGFT KLVYQNIFTA MQAMIRAMDT LKIPYKYEHN
101 KAHAQLVREV DVEKVSAFDV PDYAAIKSLW NDPGIQECYD RRREYQLSDS
151 TKYYLNDLDR VADPSYLPTQ QDVLRVRVPT TGIIEYPFDL QSVIFRMVDV
201 GGQRSERRKW IHCFENVTSI MFLVALSEYD QVLVESDNEN RMEESKALFR
```

251 TIITYPWFQN SSVILFLNKK DLLEEKIMYS HLVDYFPEYD GPQRDAQAAR

301 EFILKMFVDL NPDSDKIIYS HFTCATDTEN IRFVFAAVKD TILQLNLKEC

351 GLF*

Fig.8

RPRLSHKGPMPF

Fig. 9

QRPRLSHKGPMPF

Fig. 10

LVQPRGSRNG PGPWQGGRRK FRRQRPRLSH KGPMPF

European Patent Office

## PARTIAL EUROPEAN SEARCH REPORT

which under Rule 45 of the European Patent Convention shall be considered, for the purposes of subsequent proceedings, as the European search report

Application Number EP 03 02 0430

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y,D | TATEMOTO K ET AL: "Isolation and characterization of a novel endogenous peptide ligand for the human APJ receptor" BIOCHEMICAL AND BIOPHYSICAL RESEARCH COMMUNICATIONS, ACADEMIC PRESS INC. ORLANDO, FL, US, vol. 251, no. 2, 20 October 1998 (1998-10-20), pages 471-476, XP002101895 ISSN: 0006-291X * the whole document * ----- | 1-19 | C07K14/705 |
| Y,D | US 2002/127601 A1 (KOSTENIS EVI) 12 September 2002 (2002-09-12) * the whole document * ----- | 1-19 | |
| Y | WO 99/05177 A (CONKLIN BRUCE R ; KOSTENIS EVI (US); NAT INST HEALTH (US); UNIV CALIFO) 4 February 1999 (1999-02-04) * claims 1-25 * ----- -/-- | 1-19 | |

TECHNICAL FIELDS SEARCHED (Int.Cl.7)

C07K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 23 July 2004 | Hermann, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 03 02 0430

| Category | DOCUMENTS CONSIDERED TO BE RELEVANT<br>Citation of document with indication, where appropriate, of relevant passages | Relevant to claim | CLASSIFICATION OF THE APPLICATION (Int.Cl.7) |
|---|---|---|---|
| A,D | O'DOWD B F ET AL: "A human gene that shows identity with the gene encoding the angiotensin receptor is located on chromosome 11" GENE, ELSEVIER BIOMEDICAL PRESS. AMSTERDAM, NL, vol. 136, 1993, pages 355-360, XP002101894 ISSN: 0378-1119 * the whole document * | 1-19 | |
| X | FOLDES G ET AL: "Circulating and cardiac levels of apelin, the novel ligand of the orphan receptor APJ, in patients with heart failure" BIOCHEMICAL AND BIOPHYSICAL RESEARCH COMMUNICATIONS, ACADEMIC PRESS INC. ORLANDO, FL, US, vol. 308, no. 3, 29 August 2003 (2003-08-29), pages 480-485, XP004444564 ISSN: 0006-291X | 20-25 | TECHNICAL FIELDS SEARCHED (Int.Cl.7) |
| A | * the whole document * | 1-19 | |
| X,D | WO 03/013576 A (ANTONICEK HORST-PETER ; BAYER AG (DE); BISCHOFF ERWIN (DE); ALBRECHT B) 20 February 2003 (2003-02-20) * the whole document * | 20-25 | |
| | -/-- | | |

EPO FORM 1503 03.82 (P04C10)

European Patent Office

# PARTIAL EUROPEAN SEARCH REPORT

Application Number
EP 03 02 0430

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | CHEN MARY M ET AL: "Novel role for the potent endogenous inotrope apelin in human cardiac dysfunction." CIRCULATION. 23 SEP 2003, vol. 108, no. 12, 8 September 2003 (2003-09-08), pages 1432-1439, XP009026059 ISSN: 1524-4539 *Document accessible online September 8th 2003* * the whole document * ----- | 20-25 | |
| X | DATABASE BIOSIS 'Online! BIOSCIENCES INFORMATION SERVICE, PHILADELPHIA, PA, US; 5 November 2002 (2002-11-05), FOLDES GABOR ET AL: "Decreased cardiac and plasma levels of apelin, the novel ligand for APJ orphan receptor, in patients with heart failure." XP009030643 Database accession no. PREV200300078554 * abstract * & CIRCULATION, vol. 106, no. 19 Supplement, 5 November 2002 (2002-11-05), pages II-614, ABSTRACTS FROM SCIENTIFIC SESSIONS; CHICAGO, IL, USA; NOVEMBER 17-20, 2002 ISSN: 0009-7322 ----- | 20-25 | TECHNICAL FIELDS SEARCHED (Int.Cl.7) |

5

EPO FORM 1503 03.82 (P04C10)

European Patent Office

INCOMPLETE SEARCH SHEET C

Application Number
EP 03 02 0430

Claim(s) searched completely:
1-18

Claim(s) searched incompletely:
19

Claim(s) not searched:
-

Reason for the limitation of the search:

Present claim 19 lacks clarity (Article 84 EPC) because the method it relates to, is based on ligand for which no essential technical features are given in the claim. Thus the claim lacks clarity within the meaning of Article 84 EPC to such an extent as to render a meaningful complete search of its subject-matter impossible. Consequently, the search has been carried out for those parts of the subject-matter which can be understood in the light of the description, namely the only ligand properly defined by the description i.e. Apeline (cf. description p. 3 line 29 - p. 4 line 30, Fig. 3 and examples 2 and 3).

European Patent
Office

Application Number
EP 03 02 0430

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

European Patent Office

**LACK OF UNITY OF INVENTION SHEET B**

Application Number
EP 03 02 0430

The Search Division considers that the present European patent application does not comply with the requirements of unity of invention and relates to several inventions or groups of inventions, namely:

Invention 1: Claims 1-19

Invention 1 relates to a test system for the identification of agonist or antagonist of the APJ receptor,a method of screening for APJ receptor ligand and a medicament based on said ligand.

---

Invention 2: claims 20-25

Invention 2 relates to a method for the diagnosis or prognosis of heart diseases based on the expression monitoring of the APJ receptor and/or apelin, and kits to perform said method.

---

## ANNEX TO THE EUROPEAN SEARCH REPORT ON EUROPEAN PATENT APPLICATION NO. EP 03 02 0430

This annex lists the patent family membersrelating to the patent documents cited in the above-mentioned European search report.
The members are as contained in the European Patent Office EDP file on
The European Patent Office is in no way liable for these particulars which are merely given for the purpose of information.

23-07-2004

| Patent document cited in search report | Publication date | Patent family member(s) | Publication date |
|---|---|---|---|
| US 2002127601 A1 | 12-09-2002 | DE 10033353 A1 | 24-01-2002 |
| | | AU 7252201 A | 21-01-2002 |
| | | CA 2415460 A1 | 17-01-2002 |
| | | WO 0204665 A2 | 17-01-2002 |
| | | EP 1319022 A2 | 18-06-2003 |
| | | JP 2004502463 T | 29-01-2004 |
| | | NO 20030068 A | 07-01-2003 |
| WO 9905177 A | 04-02-1999 | WO 9905177 A1 | 04-02-1999 |
| | | US 2001053532 A1 | 20-12-2001 |
| WO 03013576 A | 20-02-2003 | DE 10138569 A1 | 30-04-2003 |
| | | CA 2456223 A1 | 20-02-2003 |
| | | WO 03013576 A1 | 20-02-2003 |
| | | EP 1416952 A1 | 12-05-2004 |

EPO FORM P0459

For more details about this annex : see Official Journal of the European Patent Office, No. 12/82